# EUROPEAN PATENT APPLICATION

(11) **EP 4 729 517 A1**
(43) Date of publication of application: **22.04.2026**
(21) Application number: 24851038.0
(22) Date of filing: 07.08.2024
(51) Int. Cl.: C07D 471/04, C07D 519/00, A61K 31/437, A61P 35/00

(54) **SPIRO COMPOUND AND USE THEREOF IN THE FIELD OF MEDICINE**

(30) Priority: 08.08.2023 CN 202310998036
(71) Applicant: Kind Pharmaceutical, Hangzhou Zhejiang 311100 (CN)
(72) Inventor: QI, Weiyi, Hangzhou, Zhejiang 311100 (CN); WU, Yunfei, Hangzhou, Zhejiang 311100 (CN); WU, Zhijie, Hangzhou, Zhejiang 311100 (CN); CHEN, Changwei, Hangzhou, Zhejiang 311100 (CN); YAN, Jian, Hangzhou, Zhejiang 311100 (CN); WANG, Shubin, Hangzhou, Zhejiang 311100 (CN); WANG, Junchao, Hangzhou, Zhejiang 311100 (CN); ZHU, Peipei, Hangzhou, Zhejiang 311100 (CN); XIONG, Min, Hangzhou, Zhejiang 311100 (CN); LIU, Dong, Hangzhou, Zhejiang 311100 (CN); DENG, Shaojiang, Hangzhou, Zhejiang 311100 (CN)
(74) Representative: Barker Brettell LLP
(86) International application number: PCT/CN2024/110366
(87) International publication number: WO 2025/031393

(57) **Abstract**

The present application relates to a spiro compound and use thereof in the field of medicine. Specifically, disclosed in the present application are a compound represented by Formula (I), an isotope-labeled compound thereof, an optical isomer thereof, a tautomer thereof, or a pharmaceutically acceptable salt thereof, or a crystal form or solvate form of the compound or pharmaceutically acceptable salt thereof.

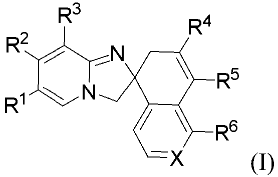

## Description

### CROSS REFERENCE TO RELATED APPLICATION

This application claims priority to the Chinese Patent Application No. 202310998036.2 filed on August 8, 2023, which is incorporated herein by reference in its entirety.

### TECHNICAL FIELD

The present disclosure relates to a class of substituted 2'H,3H-spiro[imidazo[1,2-a]pyridine-2,1'-naphthalene] compounds and their use in the field of medicine.

### BACKGROUND

Under normal physiological conditions, erythropoietin (EPO) is produced by peritubular interstitial cells in response to local tissue hypoxia. However, in renal cell carcinoma (RCC), EPO is produced by the tumor cells themselves. Statistics indicate that approximately two-thirds of RCC patients exhibit elevated EPO levels compared to normal ranges, with polycythemia occurring in about 8% of these patients (Reviews in Urology. 2002, 4(4), 163-170). In these patients, the increase in red blood cell concentration in the serum is considered to be mediated by EPO. Polycythemia vera can easily lead to venous thrombosis, bleeding, and neurovascular issues, resulting in strokes and heart diseases. Without treatment, the condition can be life-threatening. Current treatments include long-term phlebotomy, cytoreductive therapies such as hydroxyurea and interferon-alpha, as well as intravenous radioactive phosphorus, busulfan, and JAK inhibitors like ruxolitinib (Chinese Journal of Hematology. 2022, vol 43, 537-541).

Furthermore, EPO levels are generally elevated in renal cell carcinoma, making it a potential tumor biomarker. RCC patients with higher EPO levels may be more sensitive to variations in these levels. Therefore, reducing EPO levels presents a potential and effective approach for treating both polycythemia and renal cell carcinoma.

Thus, there is a need to develop an EPO inhibitor capable of significantly lowering EPO levels, enabling its application in the treatment of various diseases such as polycythemia, renal cell carcinoma, and other cancers.

### SUMMARY

The present disclosure provides a class of spirocyclic compounds and a method for their preparation, the compounds exhibiting favorable chemical stability.

The inventors have successfully prepared a novel class of spirocyclic compounds, which possess excellent chemical stability. The spirocyclic compounds provided in the present disclosure demonstrate a significant effect of reducing EPO levels in various biochemical, biological, or cellular related assays, thereby exhibiting promising application prospects in the treatment of multiple diseases such as polycythemia, renal cell carcinoma, and other cancers.

In a first aspect, the present disclosure provides a compound of formula (I), its isotopically labeled compound, its optical isomers, its tautomers, or its pharmaceutically acceptable salts, or crystalline forms or solvates of the compound or its pharmaceutically acceptable salts. The compound of formula (I), its isotopically labeled compound, its optical isomers, its tautomers, or its pharmaceutically acceptable salts thereof, or crystalline forms or solvates of the compound or its pharmaceutically acceptable salts, can be used for treating various EPO-related diseases, such as polycythemia, renal cell carcinoma, and other cancers. 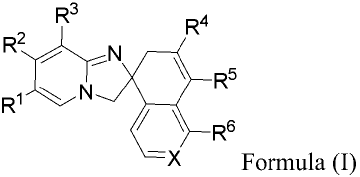 In Formula (I):
R¹ is selected from H, D, F, Cl, Br, CF₃, and CN;
R² is selected from H, D, CF₃, CH₃, OCH₃, and OH;
R³ is selected from H, D, F, Cl, Br, CF₃, CN, OCH₃, OCH₂F, OCHF₂, and OCF₃;
R⁴ is selected from H, D, F, Cl, Br, CH₃, CF₃, and CN;
R⁵ is selected from H, D, F, Cl, Br, I, CH₃, CF₃, CN, and OCH₃;
R⁶ is selected from H, D, F, Cl, Br, I, CH₃, CF₃, CN, and OCH₃; and
X is selected from N, CR⁷; wherein R⁷ is selected from H, D, F, Cl, Br, CF₃, CN, and CH₃.

Preferably, R¹ is selected from CF₃, and CN.

Preferably, R² is selected from OCH₃, and OH.

Preferably, R³ is selected from OCH₃, OCH₂F, OCHF₂, and OCF₃.

Preferably, R⁴ is selected from H, D, F, Cl, and CH₃.

Preferably, R⁵ is selected from H, D, F, Cl, CH₃, CF₃, and OCH₃.

Preferably, R⁶ is selected from F, Cl, and CH₃.

Preferably, X is selected from N, CR⁷; wherein R⁷ is selected from H, D, F, Br, CF₃, CN, and CH₃.

In some most preferred embodiments, the compound of formula (I) according to the present disclosure are selected from the various specific compounds illustrated in the examples of the present disclosure.

Unless otherwise indicated, terms such as "the compound as shown in Formula (I)," "the compound of Formula (I)," "the compound of the present disclosure," "the compound of the present disclosure," or similar terms are also intended to encompass its isotopically labeled compounds, its optical isomers, its tautomers, or its pharmaceutically acceptable salts, or crystalline forms or solvates of the compound or its pharmaceutically acceptable salts.

The term "optical isomer" refers that when a compound has one or more chiral centers, each chiral center may have an R configuration or an S configuration, and the various isomers thus constituted are known as an optical isomer. Optical isomers comprise all diastereomers, enantiomers, meso forms, racemates or mixtures thereof. For example, optical isomers can be separated by a chiral chromatography or by chiral synthesis.

The term "tautomer" refers to an isomer that is formed by rapid movement of an atom at two positions in a single molecule. It will be understood by those skilled in the art that tautomers can be mutually transformed, and in a certain state, may coexist by reaching an equilibrium state.

Unless otherwise indicated, reference to "a compound as shown by Formula (I)" or "a compound of Formula (I)" or "a compound of the disclosure" or "a compound according to the disclosure" herein also encompasses isotopically-labeled compounds obtained by replacing any atom of the compound with its isotopic atom.

Examples of isotopes suitable for inclusion in the compounds of the disclosure include isotopes of hydrogen, such as ²H (D) and ³H (T), of carbon, such as ¹¹C, ¹³C and ¹⁴C, of chlorine, such as ³⁶Cl, of fluorine, such as ¹⁸F, of iodine, such as ¹²³I and ¹²⁵I, of nitrogen, such as ¹³N and ¹⁵N, of oxygen, such as ¹⁵O, ¹⁷O and ¹⁸O, and of sulphur, such as ³⁵S.

Certain isotopically-labelled compounds of formula (I), for example, those incorporating a radioactive isotope, are useful in drug and/or substrate tissue distribution studies. The radioactive isotopes deuterium, i.e. ²H, and carbon-14, i.e. ¹⁴C, are particularly useful for this purpose in view of their ease of incorporation and ready means of detection.

Substitution with heavier isotopes such as deuterium, i.e. D, may afford certain therapeutic advantages resulting from greater metabolic stability, for example, increased in vivo half-life or reduced dosage requirements, and hence may be preferred in some circumstances. Therefore, in some embodiments, the compound of the present disclosure is an isotopically labeled compound, wherein H at each occurrence is optionally substituted with D.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds can generally be prepared by conventional techniques known to those skilled in the art or using an appropriate isotopically-labeled reagents in place of the non-labeled reagent previously employed.

The compound of the present disclosure may exist in the form of its pharmaceutically acceptable salts.

The term "pharmaceutically acceptable" means that the corresponding compound, carrier, or molecule is suitable for administration to humans. Preferably, this term refers to compounds certified by regulatory authorities such as the CFDA (China), EMEA (Europe), FDA (USA), or any other national regulatory agency for use in mammals, preferably humans.

The pharmaceutically acceptable salts of the compound of formula (I) include acid addition salts and base addition salts thereof. Suitable acid addition salts are formed from acids that form non-toxic salts. Examples include but are not limited to: acetate, adipate, aspartate, benzoate, benzenesulfonate, bicarbonate/carbonate, bisulfate/sulfate, borate, camphor sulfonate, citrate, cyclohexamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002). Methods for preparing pharmaceutically acceptable salts of the compounds described herein are known to those skilled in the art.

Moreover, the compound of the present disclosure may exist in unsolvated form as well as solvated forms with the pharmaeutially acceptable solvents such as water or ethanol. The compound of the present disclosure may also exist in one or more crystalline forms, such as polycrystalline form, or it may exist in an amorphous solid. All of these forms are included within the scope of the present disclosure.

### Preparation Method of the Compound

The compound of Formula (I) of the present disclosure can be synthesized using various methods familiar to those skilled in the art. Several exemplary synthetic methods are provided below, which are well-known in the field and can be modified in various ways as needed. If required, conventional techniques including but not limited to filtration, distillation, crystallization, column chromatography, and related techniques may be employed to isolate and purify the starting materials, intermediates, and final products. Furthermore, the specific examples provided herein also illustrate methods for synthesizing the compound of the present disclosure.

The synthetic method for the compound of the present disclosure are exemplified below using compound Ia and compound Ib as illustrative examples.

### Scheme 1:

Intermediate Ia-2 is obtained by heating Ia-1 (synthesized according to known methods or commercially available) with DAST in an organic solvent. The preferred solvent for this step is dichloromethane or dichloroethane, and the preferred reaction temperature is between 70 °C and 90 °C. Intermediate Ia-2 then reacts with a base to yield intermediate Ia-3. The preferred bases for this step are sodium hydroxide, potassium hydroxide, or cesium carbonate, and the preferred solvent for this step is methanol. Finally, intermediate Ia-3 undergoes ring closure under the action of sulfonyl chloride and a base to afford compound Ia. The preferred base for this step is triethylamine or diisopropylethylamine, and the preferred temperature is between 70 °C and 90 °C.

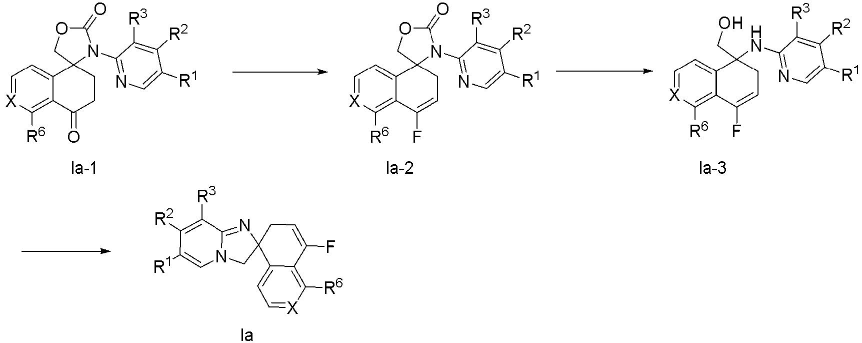

### Scheme 2:

Intermediate Ib-2 is obtained by reacting Ib-1 (synthesized according to known methods or commercially available) with a reducing agent in an organic solvent. The preferred solvent for this step is methanol or ethanol, and the preferred reaction temperature is between 0 °C and 30 °C. Intermediate Ib-2 then reacts with a dehydrating agent to yield compound Ib. The preferred dehydrating agent for this step is a Burgess reagent [methyl N-(triethylammonium sulfonyl) carbamate, CAS: 29684-56-8] or p-toluenesulfonic acid, and the preferred temperature is between 70 °C and 90 °C.

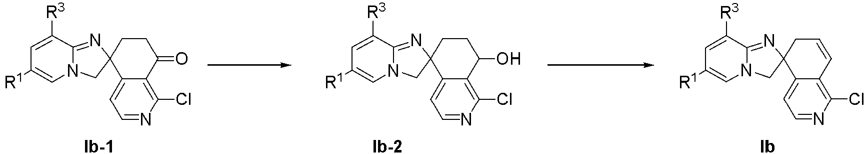

Referring to the illustrative synthetic routes for compounds Ia and Ib above, as well as the synthetic routes shown in the subsequent specific examples, those skilled in the art can synthesize other structurally similar compound of Formula (I) through appropriate modifications.

### Use of the Compound

Experimental studies have revealed that the compound of the present disclosure possesses excellent inhibitory activity against EPO. Therefore, the compound of the present disclosure can be used as EPO inhibitor drugs for treating EPO-related diseases, particularly those associated with elevated EPO levels.

In a second aspect, the present disclosure provides a pharmaceutical composition comprising the compound of the present disclosure (i.e., the compound of Formula (I)) or a pharmaceutically acceptable salt thereof, as described above, and one or more pharmaceutically acceptable carriers, adjuvants, or excipients.

The pharmaceutical composition of the present disclosure can be prepared in a manner well-known in the pharmaceutical field and can be administered via various routes, depending on whether localized or systemic treatment is desired and on the site to be treated. Administration may be local (including ocular and mucosal routes, such as intranasal, vaginal, and rectal delivery), pulmonary (e.g., via inhalation or insufflation of powders or aerosols, including via nebulizers; intratracheal, intranasal, epidermal, and transdermal), ocular, oral, or parenteral. Methods for ocular delivery may include topical administration (eye drops), subconjunctival, periocular, or intravitreal injection, or introduction via devices such as balloon catheters or ocular inserts placed in the conjunctival sac using surgical techniques. Parenteral administration includes intravenous, intraarterial, subcutaneous, intraperitoneal, or intramuscular injection or infusion; or intracranial administration (e.g., intrathecal or intraventricular). Parenteral administration may be in the form of a single bolus dose or, for example, via continuous infusion pump. Pharmaceutical compositions and formulations for topical administration may include transdermal patches, ointments, lotions, creams, gels, drops, suppositories, sprays, liquids, and powders.

If a solid carrier is used, the preparation may be tableted, placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the preparation may be in the form of a syrup, emulsion, paste, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The pharmaceutical compositions are prepared by conventional techniques appropriate to the desired preparation containing appropriate amounts of the active ingredient, that is, the compound of Formula (I) according to the present disclosure.

Compositions suitable for parenteral injection may comprise physiologically acceptable sterile aqueous or nonaqueous solutions, dispersions, suspensions or emulsions, or sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate.

These compositions may also contain excipients such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate. The following ingredients, such as (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof may also be included.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Dosage forms for topical administration of the compound of the present disclosure include paste, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

The amount of the compound of formula in the pharmaceutical composition and dosage form can be appropriately determined by those skilled in the art as needed. For example, the compound of the present disclosure can be present in the pharmaceutical composition or dosage form in a therapeutically effective amount.

In a third aspect, the present disclosure provides the compound of the present disclosure (i.e., the compound of Formula (I)) or a pharmaceutically acceptable salt thereof for use as an EPO inhibitor drug, or the use of the compound of the present disclosure (i.e., the compound of Formula (I)) or a pharmaceutically acceptable salt thereof in the manufacture of an EPO inhibitor drug.

In a fourth aspect, the present disclosure provides a method for treating an EPO-related disease or disorder (particularly one associated with elevated EPO levels), comprising administering to a patient in need thereof a therapeutically effective amount of the compound of the present disclosure (i.e., the compound of Formula (I)) or a pharmaceutically acceptable salt thereof, or the pharmaceutical composition as described above.

In a fifth aspect, the present disclosure provides the use of the compound of the present disclosure (i.e., the compound of Formula (I)) or a pharmaceutically acceptable salt thereof in the manufacture of a medicament for treating a patient suffering from an EPO-related disease or disorder (particularly one associated with elevated EPO levels).

The patient is preferably a mammal, more preferably a human patient. The route of administration during treatment may be oral, topical (including but not limited to external application, spraying, etc.), parenteral (including subcutaneous, intramuscular, intradermal, and intravenous administration), bronchial administration, nasal administration, or the like.

In some embodiments, the EPO-related disease or disorder is polycythemia, renal cell carcinoma, or other cancers.

Exemplary other cancers include bladder cancer, breast cancer, cervical cancer, colorectal cancer, small intestinal cancer, colon cancer, rectal cancer, anal cancer, endometrial cancer, head and neck cancer (e.g., cancers of larynx, laryngopharynx, nasopharynx, oropharynx, lip, and oral cavity), liver cancer (e.g., hepatocellular carcinoma, cholangiocarcinoma), lung cancer (e.g., adenocarcinoma, small cell lung cancer and non-small cell lung cancer, small cell carcinoma and non-small cell carcinoma, bronchial carcinoma, bronchial adenoma, pleuropulmonary blastoma), ovarian cancer, prostate cancer, testicular cancer, uterine cancer, esophageal cancer, gallbladder cancer, pancreatic cancer (e.g., exocrine pancreatic carcinoma), thyroid cancer, parathyroid cancer, skin cancer (e.g., squamous cell carcinoma, Kaposi's sarcoma, Merkel cell skin carcinoma), and brain cancer (e.g., astrocytoma, medulloblastoma, ependymoma, neuroectodermal tumors, pineal tumors).

In some preferred embodiments, the EPO-related disease or disorder is polycythemia.

In some preferred embodiments, the EPO-related disease or disorder is renal cell carcinoma.

A "therapeutically effective amount" of the compound of the present disclosure for treating the aforementioned diseases may be reasonably determined by an experienced physician or researcher based on factors such as the patient's condition, physical state, disease severity, route of administration, and the like.

The present disclosure will be further illustrated and described below with reference to specific examples.

### EXAMPLES

The present disclosure is further illustrated and described below with reference to specific examples. It should be understood that these examples are only for illustrating the present disclosure and not for limiting the scope of the present disclosure.

For experimental methods in the following examples where specific conditions are not indicated, they are generally carried out under the conventional conditions for such reactions, or as recommended by the manufacturers.

Unless otherwise specified, the experimental materials and reagents used in the following examples can be obtained commercially or prepared according to cited literature; the instruments and equipment used in synthesis experiments and product analysis are conventional ones commonly used in organic synthesis and analysis; the reagents, instruments, etc., for biological experiments are provided in the experimental procedures.

The following abbreviations are used herein: BINAP: 1,1'-Binaphthyl-2,2'-diyldiphenylphosphine; Cp*: Pentamethylcyclopentadienyl; DAST: Diethylaminosulfur trifluoride; DCM: Dichloromethane; DMSO: Dimethyl sulfoxide; DMA: N,N-Dimethylacetamide; DMF: N,N-Dimethylformamide; DMI: 1,3-Dimethyl-2-imidazolidinone; dppf: Diphenylphosphinoferrocene; ESI-MS: Electrospray mass spectrometry; KHMDS: Potassium hexamethyldisilazide; MsCl: Methanesulfonyl chloride; NCS: N-Chlorosuccinimide; NFSI: N-Fluorobenzenesulfonimide; NMP: N-Methyl-pyrrolidone; Pd₂dba₃: Tris(dibenzylideneacetone)dipalladium; PPTS: Pyridinium p-toluenesulfonate; Selectfluor: 1-Chloromethyl-4-fluoro-1,4-diazobicyclo[2.2.2]octane bis(tetrafluoroborate); SFC: Supercritical fluid chromatography; TBSOTf: tert-Butyl dimethylsilyl trifluoromethanesulfonate; THF: Tetrahydrofuran.

### Example 1: Preparation of 1'-Chloro-8-difluoromethoxy-8'-fluoro-6-trifluoromethyl-3H,6'H-spiro[imidazo[1,2-a]pyridine-2,5'-isoquinoline]

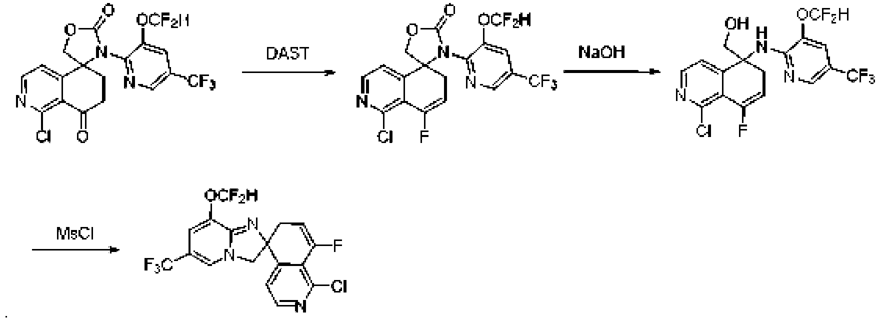

### Step 1: Synthesis of 1-Chloro-3'-(3-(difluoromethoxy)-5-(trifluoromethyl)pyridin-2-yl)-8-fluoro-6H-spiro[isoquinoline-5,4'-oxazolidin]-2'-one

1-Chloro-3'-(3-(difluoromethoxy)-5-(trifluoromethyl) pyridin-2-yl)-6,7-dihydro-8H-spiro[isoquinoline-5,4'-oxazolidin]-2',8-dione (synthesized according to WO 2021/220170, 1.01 g) was dissolved in DCM (10 mL), to which DAST (14 mL) was added at room temperature. The mixture was stirred at 80°C for 4 hours. The reaction mixture was dripped into a saturated sodium bicarbonate solution, and the resulting mixture was extracted with ethyl acetate, dried over anhydrous sodium sulfate, and purified by column chromatography to afford the target compound (79.7 mg). ESI-MS m/z: 507.28 [M+H+CH₃CN]⁺.

### Step 2: Synthesis of (1-Chloro-5-((3-(difluoromethoxy)-5-(trifluoromethyl)pyridin-2-yl)amino)-8-fluoro-5,6-dihydroisoquinolin-5-yl)methanol

The compound 1-chloro-3'-(3-(difluoromethoxy)-5-(trifluoromethyl)pyridin-2-yl)-8-fluoro-6H-spiro[isoquinoline-5,4'-oxazolidin]-2'-one (57 mg) was dissolved in MeOH (10 mL), to which 4 M NaOH (0.3 mL) was added dropwise at 0°C. The mixture was stirred at room temperature for 1 hour. The resulting mixture was extracted with ethyl acetate three times, concentrated using a rotary evaporator, and purified by preparative TLC to afford the target compound (12 mg). ESI-MS m/z: 438.29 [M-H]⁻.

### Step 3: Synthesis of 1'-Chloro-8-difluoromethoxy-8'-fluoro-6-trifluoromethyl-3H,6'H-spiro[imidazo[1,2-a]pyridine-2,5'-isoquinoline]

The compound (1-chloro-5-((3-(difluoromethoxy)-5-(trifluoromethyl)pyridin-2-yl)amino)-8-fluoro-5,6-dihydroisoquinolin-5-yl)methanol (12 mg) was dissolved in toluene (1 mL), to which MsCl (21 µL) and Et₃N (76 µL) were added. The mixture was stirred at 80°C for 1 hour. Saturated sodium bicarbonate solution was added. The resulting mixture was extracted with ethyl acetate three times, concentrated, and purified by preparative HPLC to afford the target compound, i.e., the compound of Example 1 (5.0 mg). ESI-MS m/z: 422.34 [M+H]⁺. ¹H NMR (500 MHz, DMSO-d₆) δ 8.33 (d, 1H), 7.85 (t, 1H), 7.35 (t, 1H), 7.27 (dd, 1H), 7.03 (d, 1H), 5.84 (ddd, 1H), 4.37 (d, 1H), 3.94 (d, 1H), 2.60 (ddd, 1H), 2.51 (ddd, 1H).

### Example 1-A and Example 1-B: Preparation of (S)-1'-Chloro-8-difluoromethoxy-8'-fluoro-6-trifluoromethyl-3H,6'H-spiro[imidazo[1,2-a]pyridine-2,5'-isoquinoline] and (R)-1'-Chloro-8-difluoromethoxy-8'-fluoro-6-trifluoromethyl-3H,6'H-spiro[imidazo[1,2-a]pyridine-2,5'-isoquinoline]

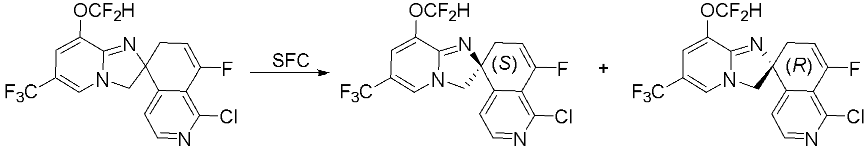

Racemic 1'-chloro-8-difluoromethoxy-8'-fluoro-6-trifluoromethyl-3H,6'H-spiro [imidazo[1,2-a]pyridine-2,5'-isoquinoline] (i.e., the compound of Example 1) was separated by preparative SFC to afford a pair of enantiomers. The enantiomer eluted first from the chiral column below was designated as Example 1-A; the later eluted enantiomer was designated as Example 1-B (Note: The absolute configurations of the compounds in Example 1-A and 1-B have not been determined. For convenience of description, the chiral center of Example 1-A is randomly assigned as S, and that of Example 1-B is assigned as R).

Example 1-A: (S)-1'-Chloro-8-difluoromethoxy-8'-fluoro-6-trifluoromethyl-3H,6'H-spiro[imidazo[1,2-a]pyridine-2,5'-isoquinoline]: SFC (RT = 4.37 min, ee = 99.7%). Analytical method: Column: DAICEL CHIRALPAK AD-H (0.46 cm x 25 cm); mobile phase: isopropanol/n-hexane (30:70 v/v); flow rate: 1 mL/min. ESI-MS m/z: 422.34 [M+H]⁺. ¹H NMR (500 MHz, DMSO-d₆) δ 8.40 (d, 1H), 7.94-7.91 (m, 1H), 7.42 (t, 1H), 7.34 (dd, 1H), 7.10 (d, 1H), 5.91 (ddd, 1H), 4.44 (d, 1H), 4.00 (d, 1H), 2.67 (ddd, 1H), 2.57 (ddd, 1H).

Example 1-B: (R)-1'-Chloro-8-difluoromethoxy-8'-fluoro-6-trifluoromethyl-3H,6'H -spiro[imidazo[1,2-a]pyridine-2,5'-isoquinoline]: SFC (RT = 4.90 min, ee = 99.3%). Analytical method: same as above. ESI-MS m/z: 422.34 [M+H]⁺. ¹H NMR (500 MHz, DMSO-d₆) δ 8.40 (d, 1H), 7.94-7.91 (m, 1H), 7.42 (t, 1H), 7.34 (dd, 1H), 7.10 (d, 1H), 5.91 (ddd, 1H), 4.44 (d, 1H), 4.01 (d, 1H), 2.67 (ddd, 1H), 2.57 (ddd, 1H).

### Example 2: Synthesis of 1'-Chloro-8-difluoromethoxy-6-trifluoromethyl-3H,6'H-spiro[imidazo[1,2-a]pyridine-2,5'-isoquinoline]

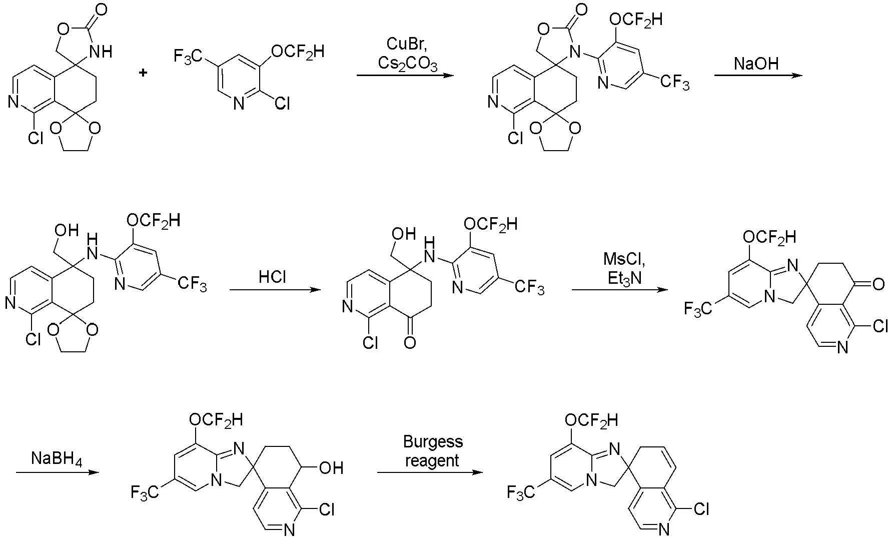

### Step 1: Synthesis of 1'-Chloro-3-(3-(difluoromethoxy)-5-(trifluoromethyl)pyridin-2-yl)-6',7'-dihydrodispiro[oxazolidine-4,5'-isoquinoline-8',2"-[1,3]dioxolane]-2-one

1'-Chloro-6',7'-dihydrodispiro[oxazolidine-4,5'-isoquinoline-8',2"-[1,3]dioxolane]-2-one (500 mg) was dissolved in DMF (10 mL), to which CuBr (240 mg), Cs₂CO₃ (1.1 g), and 2-chloro-3-(difluoromethoxy)-5-trifluoromethyl pyridine (840 mg) were added. The mixture was evacuated and backfilled with nitrogen, then stirred at 130 °C. After completion, the mixture was filtered, extracted with ethyl acetate, dried over anhydrous sodium sulfate, and concentrated using a rotary evaporator. The residue was purified by column chromatography to afford the title compound (536 mg). ESI-MS m/z: 508.23 [M+H]⁺.

### Step 2: Synthesis of (1-Chloro-5-((3-(difluoromethoxy)-5-(trifluoromethyl)pyridin-2-yl)amino)-6,7-dihydro-5H-spiro[isoquinoline-8,2'-[1,3]dioxolane]-5-yl)methanol

1'-Chloro-3-(3-(difluoromethoxy)-5-(trifluoromethyl)pyridin-2-yl)-6',7'-dihydro dispiro[oxazolidine-4,5'-isoquinoline-8',2"-[1,3]dioxolane]-2-one (150 mg) was dissolved in MeOH (3 mL), to which 4 M NaOH solution (0.7 mL) was added dropwise at 0 °C, then the mixture was stirred at room temperature for 2 h. The resulting mixture was extracted with ethyl acetate, concentrated using a rotary evaporator, and purified by preparative TLC to afford the title compound (108 mg). ESI-MS m/z: 482.18 [M+H]⁺.

### Step 3: Synthesis of (1-Chloro-5-((3-(difluoromethoxy)-5-(trifluoromethyl)pyridin-2-yl)amino)-5-(hydroxymethyl)-6,7-dihydroisoquinolin-8(5H)-one

(1-Chloro-5-((3-(difluoromethoxy)-5-(trifluoromethyl)pyridin-2-yl)amino)-6,7-dihydro-5H-spiro[isoquinoline-8,2'-[1,3]dioxolane]-5-yl)methanol (107 mg) was dissolved in THF (1 mL), to which HCl (0.4 mL) was added, and then the mixture was stirred at 50 °C for 1 h. The resulting mixture was extracted with ethyl acetate and concentrated using a rotary evaporator to afford the title compound (105 mg). ESI-MS m/z: 438.21 [M+H]⁺.

### Step 4: Synthesis of 1'-Chloro-8-(difluoromethoxy)-6-(trifluoromethyl)-6',7'-dihydro-3H,8'H-spiro[imidazo[1,2-a]pyridine-2,5'-isoquinoline]-8'-one

(1-Chloro-5-((3-(difluoromethoxy)-5-(trifluoromethyl)pyridin-2-yl)amino)-5-(hydroxymethyl)-6,7-dihydroisoquinolin-8(5H)-one (105 mg) was dissolved in toluene (5 mL) to which MsCl (186 µL) and Et₃N (675 µL) were added, and then the mixture was stirred at 80 °C. The resulting mixture was extracted with ethyl acetate, concentrated using a roatry evaporator, and purified by preparative TLC to afford the title compound (89 mg). ESI-MS m/z: 420.27 [M+H]⁺.

### Step 5: Synthesis of 1'-Chloro-8-(difluoromethoxy)-6-(trifluoromethyl)-7',8'-dihydro-3H,6'H-spiro[imidazo[1,2-a]pyridine-2,5'-isoquinoline]-8'-ol

1'-Chloro-8-(difluoromethoxy)-6-(trifluoromethyl)-6',7'-dihydro-3H,8'H-spiro[imidazo[1,2-a]pyridine-2,5'-isoquinoline]-8'-one (89 mg) was dissolved in MeOH (20 mL), to which NaBH₄ (8 mg, 1 eq.) was added at 0 °C, and then the mixture was stirred at 0 °C for 30 min. The resulting mixture was extracted with ethyl acetate, concentrated using a rotary evaporator, and purified by preparative TLC to afford the title compound (78 mg). ESI-MS m/z: 422.32 [M+H]⁺.

### Step 6: Synthesis of 1'-Chloro-8-difluoromethoxy-6-trifluoromethyl-3H,6'H-spiro[imidazo[1,2-a]pyridine-2,5'-isoquinoline]

1'-Chloro-8-(difluoromethoxy)-6-(trifluoromethyl)-7',8'-dihydro-3H,6'H-spiro[imidazo[1,2-a]pyridine-2,5'-isoquinoline]-8'-ol (45 mg) was dissolved in toluene (3 mL) to which Burgess reagent (80 mg) was added, and then the mixture was stirred at 90 °C for 30 min. The mixture was extracted with ethyl acetate, concentrated using a roatray evaporator, and purified by preparative TLC to afford the title compound (12 mg). ESI-MS m/z: 448.32 [M+HCOz]⁻. ¹H NMR (500 MHz, DMSO-d₆) δ 8.28 (d, 1H), 7.91 (s, 1H), 7.43 (t, 1H), 7.30 (d, 1H), 7.09 (d, 1H), 6.81 (d, 1H), 6.35 (dt, 1H), 4.36 (d, 1H), 3.93 (d, 1H), 2.62 (d, 2H).

### Example 2-A and Example 2-B: Preparation of (R)-1'-Chloro-8-difluoromethoxy-6-trifluoromethyl-3H,6'H-spiro[imidazo[1,2-a]pyridine-2,5'-isoquinoline] and (R)-1'-Chloro-8-difluoromethoxy-6-trifluoromethyl-3H,6'H-spiro[imidazo[1,2-a]pyridine-2,5'-isoquinoline]

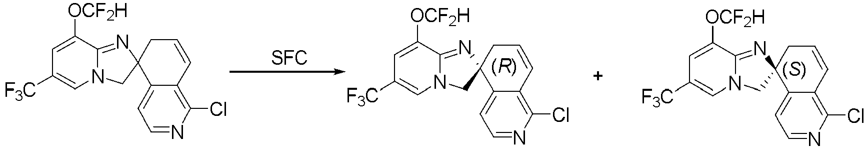

Racemic 1'-chloro-8-difluoromethoxy-6-trifluoromethyl-3H,6'H-spiro[imidazo[1,2-a]pyridine-2,5'-isoquinoline] (the compound of Example 2) was subjected to preparative SFC to obtain a pair of enantiomers. The enantiomer eluted first from the chiral column below was designated Example 2-A; and the enantiomer eluted later was designated Example 2-B. (Note: The absolute configurations of the compounds of Example 2-A and 2-B have not been determined. For narrative convenience, the chiral center of Example 2-A is randomly assigned as R, and that of Example 2-B is assigned as S.)

Example 2-A: (R)-1'-Chloro-8-difluoromethoxy-6-trifluoromethyl-3H,6'H-spiro[imidazo[1,2-a]pyridine-2,5'-isoquinoline]: SFC (RT = 5.10 min, ee = 99.6%). Analytical method: DAICEL CHIRALPAK IE-3 column (0.46 cm × 25 cm); mobile phase: isopropanol/n-hexane (30:70, v/v); flow rate: 1 mL/min. ESI-MS m/z: 404.5 [M+H]⁺; ¹H NMR (500 MHz, DMSO-d₆) δ 8.27 (d, 1H), 7.90 (s, 1H), 7.42 (t, 1H), 7.29 (dd, 1H), 7.08 (s, 1H), 6.82-6.78 (dq, 1H), 6.34 (dt, 1H), 4.35 (d, 1H), 3.92 (d, 1H), 2.63-2.60 (m, 2H).

Example 2-B: (S)-1'-Chloro-8-difluoromethoxy-6-trifluoromethyl-3H,6'H-spiro[imidazo[1,2-a]pyridine-2,5'-isoquinoline]: SFC (RT = 5.73 min, ee = 98.2%). Analytical method: DAICEL CHIRALPAK IE-3 column (0.46 cm × 25 cm); mobile phase: isopropanol/n-hexane (30:70, v/v); flow rate: 1 mL/min. ESI-MS m/z: 404.5 [M+H]⁺; ¹H NMR (500 MHz, DMSO-d₆) δ 8.27 (d, 1H), 7.91 (s, 1H), 7.42 (t, 1H), 7.29 (dd, 1H), 7.08 (s, 1H), 6.82-6.78 (dq, 1H), 6.34 (dt, 1H), 4.35 (d, 1H), 3.92 (d, 1H), 2.63-2.59 (m, 2H).

### Example 3: Synthesis of 1'-Chloro-8'-fluoro-7-methoxy-6-trifluoromethyl-3H,6'H-spiro[imidazo[1,2-a]pyridine-2,5'-isoquinoline]

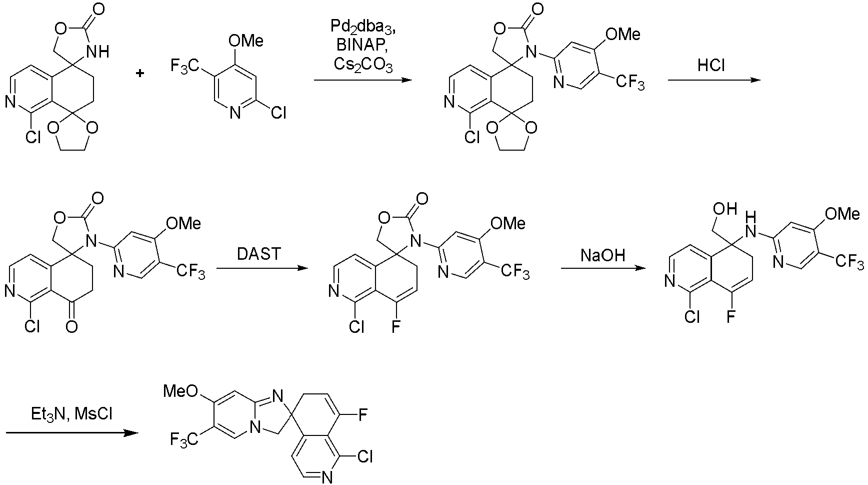

### Step 1: Synthesis of 1'-Chloro-3-(4-methoxy)-5-(trifluoromethyl)pyridin-2-yl)-6',7'-dihydrodispiro[oxazolidine-4,5'-isoquinoline-8',2"-[1,3]dioxolane]-2-one

1'-Chloro-6',7'-dihydrodispiro[oxazolidine-4,5'-isoquinoline-8',2"-[1,3]dioxolane]-2-one (1.2 g) was dissolved in toluene (15 mL), to which 2-chloro-4-methoxy-5-(trifluoromethyl)pyridine (1.0 g), Pd₂(dba)₃ (0.296 g), BINAP (0.403 g), and Cs₂CO₃ (1.97 g) were added. The mixture was evacuated and backfilled with nitrogen, then it was allowed to react at 100 °C. Water was added, and the resulting mixture was extracted with ethyl acetate, dried over anhydrous sodium sulfate, and purified by column chromatography to afford the title compound (1.14 g). ESI-MS m/z: 472.32 [M+H]⁺.

### Step 2: Synthesis of 1-Chloro-3'-(4-methoxy-5-(trifluoromethyl)pyridin-2-yl)-6,7-dihydro-8H-spiro[isoquinoline-5,4'-oxazolidine]-2',8-dione

1'-Chloro-3-(4-methoxy)-5-(trifluoromethyl)pyridin-2-yl)-6',7'-dihydrodispiro [oxazolidine-4,5'-isoquinoline-8',2"-[1,3]dioxolane]-2-one (1.14 g, 1.0 eq.) was dissolved in THF (10 mL), to which 4 N HCl (4 mL) was added. It was allowed to react at 50 °C for 3 h. The mixture was diluted with water, extracted with ethyl acetate, dried over anhydrous sodium sulfate, and concentrated to afford the title compound (1.14 g). ESI-MS m/z: 428.39 [M+H]⁺.

### Step 3: Synthesis of 1-Chloro-8-fluoro-3'-(4-methoxy-5-(trifluoromethyl)pyridin-2-yl)-6H-spiro[isoquinoline-5,4'-oxazolidine]-2'-one

1-Chloro-3'-(4-methoxy-5-(trifluoromethyl)pyridin-2-yl)-6,7-dihydro-8H-spiro[isoquinoline-5,4'-oxazolidine]-2',8-dione (1.1 g) was dissolved in DCM (10 mL), to which DAST (4.1 g) was added. It was allowed to react at 80 °C. The reaction mixture was added dropwise to saturated sodium bicarbonate solution, and the resulting mixture was extracted with ethyl acetate, dried over anhydrous sodium sulfate, and purified by column chromatography to afford the title compound (100 mg). ESI-MS m/z: 428.50 [M-H]⁻.

### Step 4: Synthesis of (1-Chloro-8-fluoro-5-((4-methoxy-5-(trifluoromethyl)pyridin-2-yl)amino)-5,6-dihydroisoquinolin-5-yl)methanol

1-Chloro-8-fluoro-3'-(4-methoxy-5-(trifluoromethyl)pyridin-2-yl)-6H-spiro[isoquinoline-5,4'-oxazolidine]-2'-one (80 mg) was dissolved in EtOH (5 mL), to which 4 N NaOH (90 µL) was added. It was allowed to react at room temperature for 1 h. The mixture was extracted with ethyl acetate, dried over anhydrous sodium sulfate, and concentrated to afford the title compound (90 mg). ESI-MS m/z: 403.32 [M+H]⁺.

### Step 5: Synthesis of 1'-Chloro-8'-fluoro-7-methoxy-6-trifluoromethyl-3H,6'H-spiro[imidazo[1,2-a]pyridine-2,5'-isoquinoline]

(1-Chloro-8-fluoro-5-((4-methoxy-5-(trifluoromethyl)pyridin-2-yl)amino)-5,6-dihydroisoquinolin-5-yl)methanol (90 mg) was dissolved in toluene (3 mL), to which MsCl (0.172 mL) and Et₃N (0.62 mL) were added. It was allowed to react at 80 °C for 2 h. The mixture was extracted with ethyl acetate and dried over anhydrous sodium sulfate. Purification by preparative TLC afforded the title compound (28 mg). ESI-MS m/z: 386.31 [M+H]⁺; ¹H NMR (500 MHz, Acetonitrile-d₃) δ 8.38 (d, J = 4.8 Hz, 1H), 8.22 (s, 1H), 7.74 (s, 1H), 7.34 (dd, 1H), 6.28 (s, 1H), 5.82 (ddd, 1H), 4.43 (d, 1H), 3.97 (d, 1H), 3.93 (s, 3H), 2.80 (ddd, 2H), 2.60 (ddd, 1H).

### Example 4: Synthesis of 1'-Chloro-8'-fluoro-6-(trifluoromethyl)-3H,6'H-spiro[imidazo[1,2-a]pyridine-2,5'-isoquinoline]-7-ol

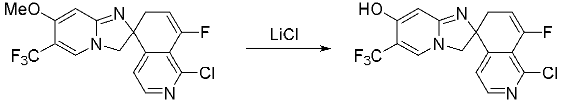

1'-Chloro-8'-fluoro-7-methoxy-6-trifluoromethyl-3H,6'H-spiro[imidazo [1,2-a]pyridine-2,5'-isoquinoline] (the compound of Example 3) (13 mg) was dissolved in DMA (3 mL), to which LiCl (21 mg) was added. It was allowed to react at 85 °C. Dilute hydrochloric acid was added, and the resulting mixture was extracted with ethyl acetate, dried over anhydrous sodium sulfate, and purified by preparative TLC to afford the title compound (10 mg). ESI-MS m/z: 371.18 [M-H]⁻; ¹H NMR (500 MHz, DMSO-d₆) δ 8.40 (d, 1H), 8.22 (s, 1H), 7.85 (s, 1H), 7.32 (dd, 1H), 5.89 (ddd, 1H), 5.32 (s, 1H), 4.36 (d, 1H), 4.15 (d, 1H), 2.71-2.66 (m, 2H).

### Example 5: Synthesis of 1'-Chloro-8-difluoromethoxy-8'-methoxy-6-trifluoromethyl-3H,6'H-spiro[imidazo[1,2-a]pyridine-2,5'-isoquinoline]

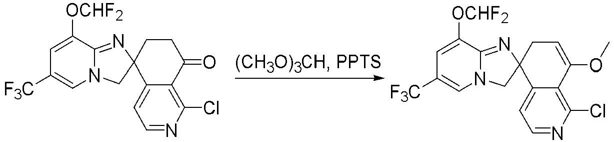

1'-Chloro-8-(difluoromethoxy)-6-(trifluoromethyl)-6',7'-dihydro-3H,8'H-spiro[imidazo[1,2-a]pyridine-2,5'-isoquinoline]-8'-one (50 mg) was dissolved in methanol (1 mL), to which PPTS (6 mg) and (CH₃O)₃CH (126 mg) were added. It was allowed to react at 120 °C for 14 hours. After cooling to room temperature, the solvent was removed using a rotary evaporator. Purification by preparative TLC afforded the title compound (11.3 mg). ESI-MS m/z: 478.31 [M-H+CH₃CN]⁻; ¹H NMR (500 MHz, DMSO-d₆) δ 8.33 (d, 1H), 7.90 (s, 1H), 7.45 (t, 1H), 7.28 (d, 1H), 7.09 (s, 1H), 5.31 (dd, 1H), 4.46 (d, 1H), 3.88 (d, 1H), 3.70 (s, 3H), 2.57 (dd, 1H), 2.48-2.43 (m, 1H).

### Example 6: Synthesis of 1'-Chloro-8-difluoromethoxy-8'-methyl-6-trifluoromethyl-3H,6'H-spiro[imidazo[1,2-a]pyridine-2,5'-isoquinoline]

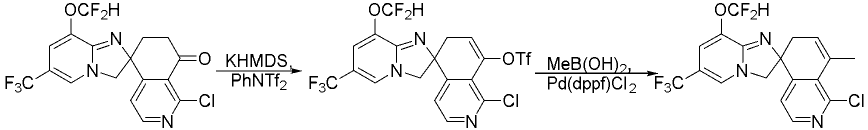

### Step 1: Synthesis of 1'-Chloro-8-difluoromethoxy-6-trifluoromethyl-3H,6'H-spiro[imidazo[1,2-a]pyridine-2,5'-isoquinoline]-8'-yl trifluoromethanesulfonate

1'-Chloro-8-(difluoromethoxy)-6-(trifluoromethyl)-6',7'-dihydro-3H,8'H-spiro[imidazo[1,2-a]pyridine-2,5'-isoquinoline]-8'-one (300 mg) was dissolved in THF (6 mL) and cooled to -78 °C, to which KHMDS (0.858 mL) was added dropwise. After 0.5 h, PhNTf₂ (306 mg) was dissolved in THF (1 mL) and then was added dropwise to the reaction mixture. It was allowed to react for 1 h. Saturated NH₄Cl (2 mL) was added, and the resulting mixture was extracted, and the product was isolated by column chromatography to afford the title compound (195 mg).

### Step 2: Synthesis of 1'-Chloro-8-difluoromethoxy-8'-methyl-6-trifluoromethyl-3H,6'H-spiro[imidazo[1,2-a]pyridine-2,5'-isoquinoline]

1'-Chloro-8-difluoromethoxy-6-trifluoromethyl-3H,6'H-spiro[imidazo[1,2-a]pyridine-2,5'-isoquinoline]-8'-yl trifluoromethanesulfonate (20 mg), MeB(OH)₂ (6.5 mg), K3PO₄ (23 mg), Pd(dppf)Cl₂ (5 mg), 1,4-dioxane (1 mL), and H₂O (1 mL) were added to a reaction tube. After nitrogen purging, it was allowed to react at 70 °C for 1 h. The mixture was extracted, concentrated by a rotary evaporator for removal of solvent and purified by preparative TLC to afford the title compound (10 mg). ESI-MS m/z: 418.32 [M+H]⁺; ¹H NMR (500 MHz, DMSO-d₆) δ 8.29 (d, 1H), 7.90 (s, 1H), 7.44 (t, 1H), 7.30 (s, 1H), 7.10 (s, 1H), 6.15 (td, 1H), 4.40 (s, 1H), 3.85 (s, 1H), 2.46 (s, 2H), 2.31 (dd, 3H).

### Example 7: Synthesis of 1',8'-Dichloro-8-difluoromethoxy-6-trifluoromethyl-3H,6'H-spiro[imidazo[1,2-a]pyridine-2,5'-isoquinoline]

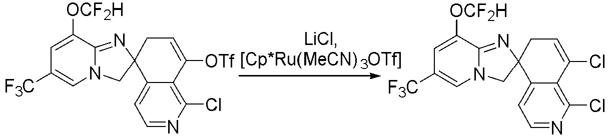

1'-Chloro-8-difluoromethoxy-6-trifluoromethyl-3H,6'H-spiro[imidazo[1,2-a]pyridine-2,5'-isoquinoline]-8'-yl trifluoromethanesulfonate (50 mg), LiCl (1.2 mg), [Cp*Ru(MeCN)₃OTf] (0.6 mg), and NMP (1 mL) were added to a reaction tube. After nitrogen purging, it was allowed to react at 100 °C for 3 h. The mixture was extracted, concentrated by a rotary evaporator for removal of solvent and purified by preparative TLC to afford the title compound (5.2 mg). ESI-MS m/z: 438.19 [M+H]⁺; ¹H NMR (500 MHz, DMSO-d₆) δ 8.40 (d, 1H), 7.94 (s, 1H), 7.42 (t, 1H), 7.36 (d, 1H), 7.12 (s, 1H), 6.59 (t, 1H), 4.45 (d, 1H), 4.02 (d, 1H), 2.62 (d, 2H).

### Example 8: Synthesis of 1'-Chloro-8-difluoromethoxy-6,8'-bis(trifluoromethyl)-3H,6'H-spiro[imidazo[1,2-a]pyridine-2,5'-isoquinoline]

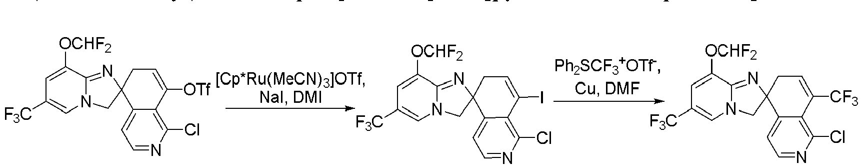

### Step 1: Synthesis of 1'-Chloro-8-difluoromethoxy-8'-iodo-6-trifluoromethyl-3H,6'H-spiro[imidazo[1,2-a]pyridine-2,5'-isoquinoline]

1'-Chloro-8-difluoromethoxy-6-trifluoromethyl-3H,6'H-spiro[imidazo[1,2-a]pyridine-2,5'-isoquinoline]-8'-yl trifluoromethanesulfonate (75 mg), NaI (105 mg), [Cp*Ru(MeCN)₃OTf] (14.2 mg) and DMI (5 mL) were added to a reaction tube. After nitrogen purging, it was allowed to react at 100 °C for 12 h. The mixture was extracted, concentrated by a rotary evaporator for removal of solvent and purified by preparative TLC to afford the title compound (52.6 mg).

### Step 2: Synthesis of 1'-Chloro-8-difluoromethoxy-6,8'-bis(trifluoromethyl)-3H,6'H-spiro[imidazo[1,2-a]pyridine-2,5'-isoquinoline]

1'-Chloro-8-difluoromethoxy-8'-iodo-6-trifluoromethyl-3H,6'H-spiro[imidazo[1,2-a]pyridine-2,5'-isoquinoline] (30.0 mg, 1.0 eq.), copper powder (25.4 mg), and diphenyl(trifluoromethyl)sulfonium trifluoromethanesulfonate (57.8 mg) were dissolved in DMF (2.0 mL). After nitrogen purging, it was allowed to react at 100 °C for 2 h. The mixture was extracted, concentrated by a rotary evaporator for removal of solvent and purified by preparative TLC to afford the title compound (0.9 mg). ESI-MS m/z: 472.30 [M+H]⁺; ¹H NMR (500 MHz, Acetonitrile-d₃) δ 8.38 (d, 1H), 7.55 (t, 1H), 7.39 (d, 1H), 7.24-7.20 (m, 1H), 7.20 (t, 1H), 6.99 (d, 1H), 4.34 (d, 1H), 3.90 (d, 1H), 2.78-2.71 (m, 1H), 2.70-2.63 (m, 1H).

### Example 9: Synthesis of 1',7'-Dichloro-8-difluoromethoxy-6-trifluoromethyl-3H,6'H-spiro[imidazo[1,2-a]pyridine-2,5'-isoquinoline]

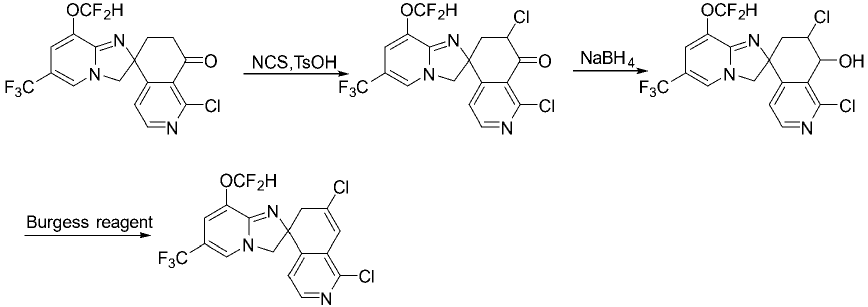

### Step 1: Synthesis of 1',7'-Dichloro-8-difluoromethoxy-6-trifluoromethyl-6',7'-dihydro-3H,8'H-spiro[imidazo[1,2-a]pyridine-2,5'-isoquinoline]-8'-one

1'-Chloro-8-(difluoromethoxy)-6-(trifluoromethyl)-6',7'-dihydro-3H,8'H-spiro[imidazo[1,2-a]pyridine-2,5'-isoquinoline]-8'-one (127 mg), TsOH (52 mg), NCS (61 mg), and CH₃CN (4 mL) were added to a reaction flask and it was allowed to react at 50 °C for 12 h. The reaction mixture was concentrated, and purified by preparative TLC to afford the title compound (36 mg).

### Step 2: Synthesis of 1',7'-Dichloro-8-difluoromethoxy-6-trifluoromethyl-7',8'-dihydro-3H,6'H-spiro[imidazo[1,2-a]pyridine-2,5'-isoquinoline]-8'-ol

1',7'-Dichloro-8-difluoromethoxy-6-trifluoromethyl-6',7'-dihydro-3H,8'H-spiro[imidazo[1,2-a]pyridine-2,5'-isoquinoline]-8'-one (36 mg) was dissolved in MeOH (2 mL, to which NaBH₄ (3 mg) was added under an ice bath, and it was allowed to react for 0.5 h. Water was added, and the resulting mixture was extracted, and purified by preparative TLC to afford the title compound (20 mg).

### Step 3: Synthesis of 1',7'-Dichloro-8-difluoromethoxy-6-trifluoromethyl-3H,6'H-spiro[imidazo[1,2-a]pyridine-2,5'-isoquinoline]

1',7'-Dichloro-8-difluoromethoxy-6-trifluoromethyl-7',8'-dihydro-3H,6'H-spiro[imidazo[1,2-a]pyridine-2,5'-isoquinoline]-8'-ol (20 mg) was dissolved in 1,4-dioxane (3 mL), to which Burgess reagent (31 mg) was added. It was allowed to react at 100 °C for 1 h. Water was added, and the mixture was extracted, and purified by preparative TLC to afford the title compound (0.73 mg). ESI-MS m/z: 438.18 [M+H]⁺; ¹H NMR (500 MHz, CD₃CN) δ 8.16 (d, 1H), 7.46 (t, 1H), 7.18 (d, 1H), 7.06 (t, 1H), 6.93 (d, 1H), 6.87 (d, 1H), 4.25 (d, 1H), 3.82 (d, 1H), 2.98 (dd, 1H), 2.69 (d, 1H).

### Example 10: Synthesis of 1'-Chloro-8-difluoromethoxy-7'-fluoro-6-trifluoromethyl-3H,6'H-spiro[imidazo[1,2-a]pyridine-2,5'-isoquinoline]

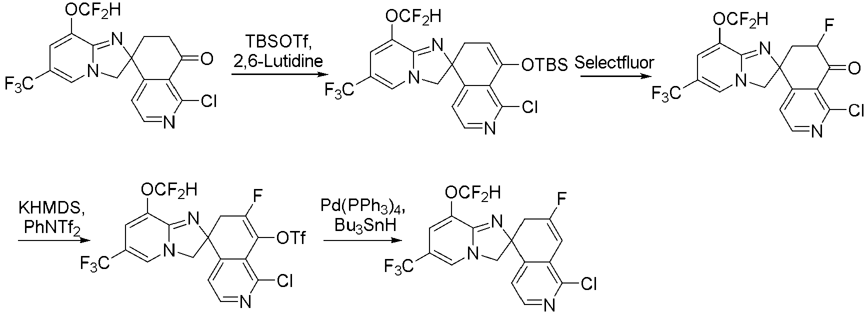

### Step 1: Synthesis of 8'-((tert-Butyldimethylsilyl)oxy)-1'-chloro-8-difluoromethoxy-7'-fluoro-6-trifluoromethyl-3H,6'H-spiro[imidazo[1,2-a]pyridine-2,5'-isoquinoline]

1'-Chloro-8-(difluoromethoxy)-6-(trifluoromethyl)-6',7'-dihydro-3H,8'H-spiro[imidazo[1,2-a]pyridine-2,5'-isoquinoline]-8'-one (200 mg) and 2,6-dimethylpyridine (257 mg) were dissolved in DCM (10 mL). After nitrogen purging, TBSOTf (380 mg) was added under an ice bath. It was allowed to react at room temperature for 10 min, then at 40 °C for 2 h. Water was added, and the mixture was extracted, concentrated, and purified by column chromatography to afford the title compound (221.6 mg).

### Step 2: Synthesis of 1'-Chloro-8-difluoromethoxy-7'-fluoro-6-trifluoromethyl-6',7'-dihydro-3H,8'H-spiro[imidazo[ 1,2-a]pyridine-2,5'-isoquinoline]-8'-one

8'-((tert-Butyldimethylsilyl)oxy)-1'-chloro-8-difluoromethoxy-7'-fluoro-6-trifluoromethyl-3H,6'H-spiro[imidazo[1,2-a]pyridine-2,5'-isoquinoline] (221.6 mg) was dissolved in MeCN (10 mL) and cooled to 0 °C, to which Selectfluor (159.4 mg) was added, and it was allowed to react at room temperature for 3 h. Water was added, and the mixture was extracted, concentrated using a rotary evaporator, and purified by column chromatography to afford the title compound (75.6 mg).

### Step 3: Synthesis of 1'-Chloro-8-difluoromethoxy-7'-fluoro-6-trifluoromethyl-3H,6'H-spiro[imidazo[1,2-a]pyridine-2,5'-isoquinoline]-8'-yl trifluoromethanesulfonate

1'-Chloro-8-difluoromethoxy-7'-fluoro-6-trifluoromethyl-6',7'-dihydro-3H,8'H-spiro[imidazo[1,2-a]pyridine-2,5'-isoquinoline]-8'-one (40.0 mg) was dissolved in THF (8 mL) and cooled to -78 °C, to which KHMDS (0.11 mL) was added dropwise, and the mixture was stirred at -78 °C for 0.5 h. PhNTf₂ (48.6 mg) was added, and the resulting mixture was stirred at -78 °C for 2 h. The reaction was quenched, and the mixture was concentrated using a rotary evaporator, and purified by preparative TLC to afford the title compound (15.4 mg).

### Step 4: Synthesis of 1'-Chloro-8-difluoromethoxy-7'-fluoro-6-trifluoromethyl-3H,6'H-spiro[imidazo[1,2-a]pyridine-2,5'-isoquinoline]

1'-Chloro-8-difluoromethoxy-7'-fluoro-6-trifluoromethyl-3H,6'H-spiro[imidazo[1,2-a]pyridine-2,5'-isoquinoline]-8'-yl trifluoromethanesulfonate (15.4 mg), Pd(PPh₃)₄ (3.1 mg), and LiCl (9.3 mg) were dissolved in THF (2 mL). After nitrogen purging, Bu₃SnH (11.9 mg) was added, and it was allowed to react at room temperature for 3 h. The mixture was extracted, concentrated using a rotary evaporator, and purified by preparative TLC to afford the title compound (2.0 mg). ESI-MS m/z: 422.26 [M+H]⁺; ¹H NMR (500 MHz, CD₃CN) δ 8.23 (d, 1H), 7.59 (p, 1H), 7.27 (dd, J = 5.0, 0.7 Hz, 1H), 7.18 (t, 1H), 7.00 (d, 1H), 6.52 (ddd, 1H), 4.41 (d, 1H), 3.96 (d, 1H), 3.04 (dt, 1H), 2.73 (dd, 1H).

### Example 10-A and Example 10-B: Preparation of (R)-1'-Chloro-8-difluoromethoxy-7'-fluoro-6-trifluoromethyl-3H,6'H-spiro[imidazo[1,2-a]pyridine-2,5'-isoquinoline] and (S)-1'-Chloro-8-difluoromethoxy-7'-fluoro-6-trifluoromethyl-3H,6'H-spiro[imidazo[1,2-a]pyridine-2,5'-isoquinoline]

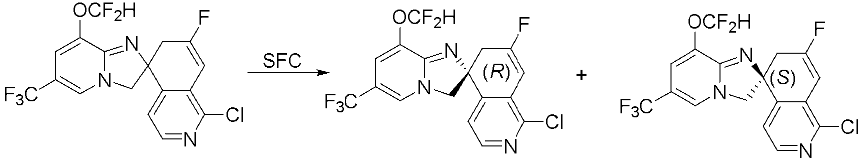

Racemic 1'-chloro-8-difluoromethoxy-7'-fluoro-6-trifluoromethyl-3H,6'H-spiro[imidazo[1,2-a]pyridine-2,5'-isoquinoline] (the compound of Example 10) was subjected to preparative SFC to obtain a pair of enantiomers. The enantiomer eluted first from the chiral column below was designated Example 10-A; and the enantiomer eluted later was designated Example 10-B. (Note: The absolute configurations of the compounds of Example 10-A and 10-B have not been determined. For narrative convenience, the chiral center of Example 10-A is randomly assigned as R, and that of Example 10-B is assigned as S.)

Example 10-A: (R)-1'-Chloro-8-difluoromethoxy-7'-fluoro-6-trifluoromethyl-3H,6'H-spiro[imidazo[1,2-a]pyridine-2,5'-isoquinoline]: SFC (RT = 2.81 min, ee = 99.8%). Analytical method: DAICEL CHIRALPAK IE column (0.46 cm × 25 cm); mobile phase: isopropanol/n-hexane (20:80, v/v); flow rate: 1 mL/min. ESI-MS m/z: 422.4 [M+H]⁺; ¹H NMR (500 MHz, DMSO-d₆) δ 8.26 (d, 1H), 7.96 (s, 1H), 7.39 (t, 1H), 7.31 (dd, 1H), 7.11 (s, 1H), 6.46 (dd, 1H), 4.44 (d, 1H), 4.12 (d, 1H), 3.00 (dd, 1H), 2.89 (dd, 1H).

Example 10-B: (S)-1'-Chloro-8-difluoromethoxy-7'-fluoro-6-trifluoromethyl-3H,6'H-spiro[imidazo[1,2-a]pyridine-2,5'-isoquinoline]: SFC (RT = 3.68 min, ee = 99.9%). Analytical method: DAICEL CHIRALPAK IE column (0.46 cm × 25 cm); mobile phase: isopropanol/n-hexane (20:80, v/v); flow rate: 1 mL/min. ESI-MS m/z: 422.4 [M+H]⁺; ¹H NMR (500 MHz, DMSO-d₆) δ 8.26 (d, 1H), 7.96 (s, 1H), 7.39 (t, 1H), 7.31 (dd, 1H), 7.11 (s, 1H), 6.46 (dd, 1H), 4.44 (d, 1H), 4.11 (d, 1H), 3.00 (dd, 1H), 2.89 (dd, 1H).

### Example 11: Synthesis of 6'-Bromo-8-difluoromethoxy-4',5'-difluoro-6-trifluoromethyl-2'H,3H-spiro[imidazo[1,2-a]pyridine-2,1'-naphthalene]

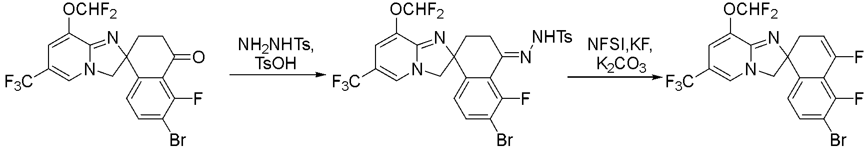

### Step 1: Synthesis of N'-(6'-Bromo-8-difluoromethoxy-5'-fluoro-6-trifluoromethyl-2',3'-dihydro-3H,4'H-spiro[imidazo[1,2-a]pyridine-2,1'-naphthalene]-4'-ylidene)-4-toluenesulfonylhydrazone

6'-Bromo-8-difluoromethoxy-5'-fluoro-6-trifluoromethyl-2',3'-dihydro-3'H,4'H-spiro[imidazo[1,2-a]pyridine-2,1'-naphthalene]-4'-one (synthesis steps refer to Example 2, Steps 1-4) (200 mg), TsNHNH₂ (110 mg), and TsOH (10 mg) were added to MeOH (5 mL) and it was allowed to react at 80 °C for 12 h. The mixture was extracted, concentrated, and purified by preparative TLC to afford the title compound (140 mg).

### Step 2: Synthesis of 6'-Bromo-8-difluoromethoxy-4',5'-difluoro-6-trifluoromethyl-2'H,3H-spiro[imidazo[1,2-a]pyridine-2,1'-naphthalene]

N'-(6'-Bromo-8-difluoromethoxy-5'-fluoro-6-trifluoromethyl-2',3'-dihydro-3H,4'H-spiro[imidazo[1,2-a]pyridine-2,1'-naphthalene]-4'-ylidene)-4-toluene sulfonylhydrazone (45 mg), NFSI (32.7 mg), K₂CO₃ (11.6 mg), and KF (27.6 mg) were added to Dioxane (2 mL) and it was allowed to react at 80 °C for 12 h. Water was added, and the mixture was extracted, concentrated using a rotary evaporator, and purified by preparative TLC to afford the title compound (3.1 mg). ESI-MS m/z: 483.20, 485.20 [M+H]⁺; ¹H NMR (500 MHz, DMSO-d₆) δ 7.90 (d, 1H), 7.72 (dd, 1H), 7.42 (t, 1H), 7.08 (m, 2H), 5.79 (ddd, 1H), 4.40 (d, 1H), 3.93 (d, 1H), 2.67 (dd, 1H), 2.58-2.52 (m, 1H).

### Example 12: Synthesis of 6'-Bromo-8-(difluoromethoxy)-5'-fluoro-6-(trifluoromethyl)-2'H,3H-spiro[imidazo[1,2-a]pyridine-2,1'-naphthalene]

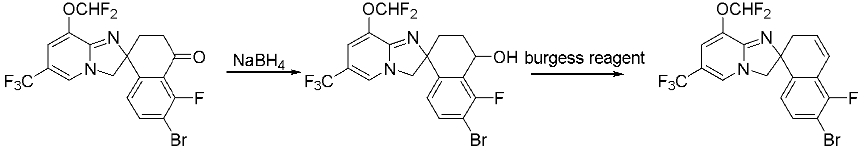

### Step 1: Synthesis of 6'-Bromo-8-(difluoromethoxy)-5'-fluoro-6-(trifluoromethyl)-3',4'-dihydro-2'H,3H-spiro[imidazo[1,2-a]pyridine-2,1'-naphthalene]-4'-ol

6'-Bromo-8-difluoromethoxy-5'-fluoro-6-trifluoromethyl-2',3'-dihydro-3'H,4'H-spiro[imidazo[1,2-a]pyridine-2,1'-naphthalene]-4'-one (60 mg) was dissolved in MeOH (2 mL), to which NaBH₄ (2 mg) was added, and it was allowe to react at room temperature for 0.5 h. Water was added, and the mixture was extracted, concentrated using a rotary evaporator, and purified by preparative TLC to afford the title compound (31 mg).

### Step 2: Synthesis of 6'-Bromo-8-difluoromethoxy-5'-fluoro-6-trifluoromethyl-2'H,3H-spiro[imidazo[1,2-a]pyridine-2,1'-naphthalene]

6'-Bromo-8-difluoromethoxy-5'-fluoro-6-trifluoromethyl-3',4'-dihydro-2'H,3H-spiro[imidazo[1,2-a]pyridine-2,1'-naphthalene]-4'-ol (31 mg) and Burgess reagent (46 mg) were dissolved in toluene (2 mL) and it was allowed to react at 90 °C for 5 h. Water was added, and the mixture was extracted, concentrated using a rotary evaportor, and purified by preparative HPLC to afford the title compound (5.6 mg). ESI-MS m/z: 465.07, 467.14 [M+H]⁺; ¹H NMR (500 MHz, DMSO-d₆) δ 7.89 (t, 1H), 7.56 (dd, 1H), 7.43 (t, 1H), 7.05 (s, 1H), 6.75 (d, 1H), 6.24 (dt, 1H), 4.31 (d, 1H), 3.84 (d, 1H), 2.59-2.55 (m, 2H).

### Example 13: Synthesis of 6'-Bromo-8-difluoromethoxy-5'-fluoro-4'-iodo-6-trifluoromethyl-2'H,3H-spiro[imidazo[1,2-a]pyridine-2,1'-naphthalene]

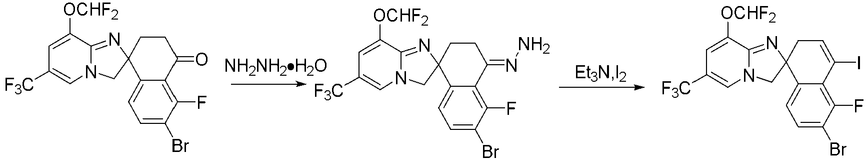

### Step 1: Synthesis of 6'-Bromo-8-difluoromethoxy-5'-fluoro-4'-hydrazinylidene-6-trifluoromethyl-3',4'-dihydro-2'H,3H-spiro[imidazo[1,2-a]pyridine-2,1'-naphthalene]

6'-Bromo-8-difluoromethoxy-5'-fluoro-6-trifluoromethyl-2',3'-dihydro-3'H,4'H-spiro[imidazo[1,2-a]pyridine-2,1'-naphthalene]-4'-one (40 mg), hydrazine hydrate (2 mL), and EtOH (2 mL) were added to a reaction tube and it was allowed to react at 80 °C for 1 h. Water was added, and the mixture was extracted, and concentrated to afford the title compound (40 mg).

### Step 2: Synthesis of 6'-Bromo-8-difluoromethoxy-5'-fluoro-4'-iodo-6-trifluoromethyl-2'H,3H-spiro[imidazo[1,2-a]pyridine-2,1'-naphthalene]

6'-Bromo-8-difluoromethoxy-5'-fluoro-4'-hydrazinylidene-6-trifluoromethyl-3',4'-dihydro-2'H,3H-spiro[imidazo[1,2-a]pyridine-2,1'-naphthalene] (45 mg), I₂ (25 mg), and Et₃N (25 mg) were added to THF (2 mL) and it was allowed to react at room temperature for 0.5 h. Water was added, and the mixture was extracted, concentrated using a rotary evaporator, and purified by preparative TLC to afford the title compound (2.5 mg). ESI-MS m/z: 591.02, 592.84 [M+H]⁺; ¹H NMR (500 MHz, DMSO-d₆) δ 7.91 (q, 1H), 7.69 (dd, 1H), 7.42 (t, 1H), 7.08-7.04 (m, 3H), 4.41 (d, 1H), 3.88 (d, 1H), 2.57-2.52 (m, 1H), 2.41 (dd, 1H).

### Example 14: Synthesis of 6'-Bromo-4'-chloro-8-difluoromethoxy-5'-fluoro-4'-iodo-6-trifluoromethyl-2'H,3H-spiro[imidazo[1,2-a]pyridine-2,1'-naphthalene]

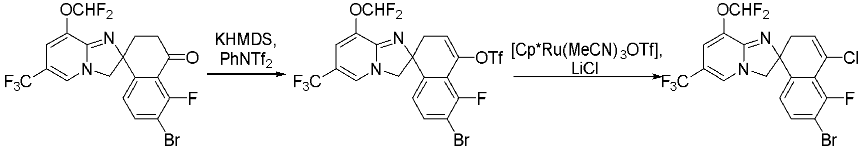

### Step 1: Synthesis of 6'-Bromo-8-difluoromethoxy-5'-fluoro-6-trifluoromethyl-2'H,3H-spiro[imidazo[1,2-a]pyridine-2,1'-naphthalene]-4'-yl trifluoromethanesulfonate

6'-Bromo-8-difluoromethoxy-5'-fluoro-6-trifluoromethyl-2',3'-dihydro-3'H,4'H-spiro[imidazo[1,2-a]pyridine-2,1'-naphthalene]-4'-one (200 mg) was dissolved in THF (5 mL) and cooled to -78 °C, to which KHMDS (0.5 mL) was added dropwise. After 0.5 h, PhNTf₂ (178 mg) was added, and it was allowed to react for 1 h. The reaction was quenched with saturated NH₄Cl solution, and the resulting mixture was extracted, concentrated using a rotary evaporator, and purified by silica gel column chromatography to afford the title compound (145 mg).

### Step 2: Synthesis of 6'-Bromo-4'-chloro-8-(difluoromethoxy)-5'-fluoro-4'-iodo-6-trifluoromethyl-2'H,3H-spiro[imidazo[1,2-a]pyridine-2,1'-naphthalene]

6'-Bromo-8-difluoromethoxy-5'-fluoro-6-trifluoromethyl-2'H,3H-spiro [imidazo[1,2-a]pyridine-2,1'-naphthalene]-4'-yl trifluoromethanesulfonate (30 mg), LiCl (3.1 mg), and [Cp*Ru(MeCN)₃OTf] (1.2 mg) were added to NMP (2 mL). After nitrogen purging, it was allowed to react at 100 °C for 3 h. Water was added, and the mixture was extracted, concentrated using a rotary evaporator, and purified by preparative TLC to afford the title compound (14.5 mg). ESI-MS m/z: 499.14, 500.69 [M+H]⁺; ¹H NMR (500 MHz, DMSO-d₆) δ 7.90 (t, 1H), 7.73 (dd, 1H), 7.42 (t, 1H), 7.10 (d, 1H), 7.07 (d, 1H), 6.43 (dd, 1H), 4.40 (d, 1H), 3.92 (d, 1H), 2.66-2.55 (m, 2H).

### Example 15: Synthesis of 8-(Difluoromethoxy)-4',5'-difluoro-6-(trifluoromethyl)-2'H,3H-spiro[imidazo[1,2-a]pyridine-2,1'-naphthalene]

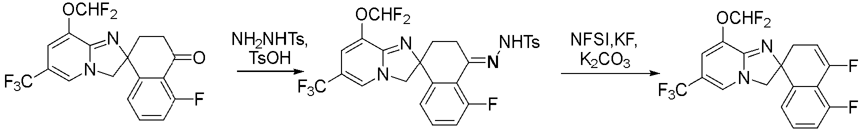

### Step 1: Synthesis of N'-(8-Difluoromethoxy-5'-fluoro-6-trifluoromethyl-2',3'-dihydro-3H,4'H-spiro[imidazo[1,2-a]pyridine-2,1'-naphthalene]-4'-ylidene)-4-toluene sulfonylhydrazone

8-Difluoromethoxy-5'-fluoro-6-trifluoromethyl-2',3'-dihydro-3'H,4'H-spiro[imidazo[1,2-a]pyridine-2,1'-naphthalene]-4'-one (150 mg), TsNHNH₂ (104 mg), and TsOH (2 mg) were added to MeOH (5 mL) and it was allowed to react at 80 °C for 12 h. The mixture was extracted, concentrated, and purified by preparative TLC to afford the title compound (55 mg).

### Step 2: Synthesis of 8-Difluoromethoxy-4',5'-difluoro-6-trifluoromethyl-2'H,3H-spiro[imidazo[1,2-a]pyridine-2,1'-naphthalene]

N'-(8-Difluoromethoxy-5'-fluoro-6-trifluoromethyl-2',3'-dihydro-3H,4'H-spiro[imidazo[1,2-a]pyridine-2,1'-naphthalene]-4'-ylidene)-4-toluenesulfonylhydrazone (45 mg), NFSI (37 mg), K₂CO₃ (32.6 mg), and KF (13.7 mg) were added to Dioxane (2 mL) and it was allowed to react at 80 °C for 12 h. Water was added, and the mixture was extracted, concentrated using a rotary evaporator, and purified by preparative TLC to afford the title compound (3.1 mg). ESI-MS m/z: 405.00 [M+H]⁺; ¹H NMR (500 MHz, DMSO-d₆) δ 7.90 (d, 1H), 7.72 (dd, 1H), 7.42 (t, 1H), 7.08 (m, 2H), 5.79 (ddd, 1H), 4.40 (d, 1H), 3.93 (d, 1H), 2.67 (dd, 1H), 2.58-2.52 (m, 1H).

### Example 15-A and Example 15-B: Preparation of (S)-8-Difluoromethoxy-4',5'-difluoro-6-trifluoromethyl-2'H,3H-spiro[imidazo[1,2-a]pyridine-2,1'-naphthalene] and (R)-8-Difluoromethoxy-4',5'-difluoro-6-trifluoromethyl-2'H,3H-spiro[imidazo[1,2-a]pyridine-2,1'-naphthalene]

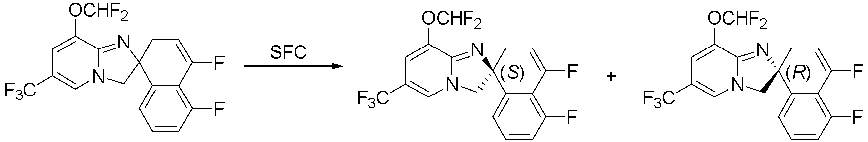

Racemic 8-difluoromethoxy-4',5'-difluoro-6-trifluoromethyl-2'H,3H-spiro[imidazo[1,2-a]pyridine-2,1'-naphthalene] (the compound of Example 15) was subjected to preparative SFC to obtain a pair of enantiomers. The enantiomer eluted first from the chiral column below was designated Example 15-A; the enantiomer eluted later was designated Example 15-B. (Note: The absolute configurations of the compounds of Example 15-A and 15-B have not been determined. For narrative convenience, the chiral center of Example 15-A is randomly assigned as S, and that of Example 15-B is assigned as R.)

Example 15-A: (S)-8-Difluoromethoxy-4',5'-difluoro-6-trifluoromethyl-2'H,3H-spiro[imidazo[1,2-a]pyridine-2,1'-naphthalene]: SFC (RT = 8.49 min, ee = 99.9%). Analytical method: DAICEL CHIRALPAK IG column (0.46 cm × 25 cm); mobile phase: ethanol/n-hexane (9:95, v/v) with 0.1% diethylamine; flow rate: 1 mL/min. ESI-MS m/z: 405.5 [M+H]⁺; ¹H NMR (500 MHz, DMSO-d₆) δ 7.90 (s, 1H), 7.43 (t, 1H), 7.41 (dt, 1H), 7.23-7.17 (m, 1H), 7.13 (dd, 1H), 7.06 (s, 1H), 5.69 (ddd, 1H), 4.41 (d, 1H), 3.90 (d, 1H), 2.65 (ddd, 1H), 2.56-2.52 (m, 1H).

Example 15-B: (R)-8-Difluoromethoxy-4',5'-difluoro-6-trifluoromethyl-2'H,3H-spiro[imidazo[1,2-a]pyridine-2,1'-naphthalene]: SFC (RT = 9.88 min, ee = 99.3%). Analytical method: DAICEL CHIRALPAK IG column (0.46 cm × 25 cm); mobile phase: ethanol/n-hexane (5:95, v/v) with 0.1% diethylamine; flow rate: 1 mL/min. ESI-MS m/z: 405.5 [M+H]⁺; ¹H NMR (500 MHz, DMSO-d₆) δ 7.89 (s, 1H), 7.43 (t, 1H), 7.42 (dt, 1H), 7.19 (dd, 1H), 7.13 (dd, 1H), 7.05 (s, 1H), 5.69 (ddd, 1H), 4.41 (d, 1H), 3.90 (d, 1H), 2.65 (ddd, 1H), 2.56-2.52 (m, 1H).

### Example 16: Synthesis of 8-(Difluoromethoxy)- 4',5'-fluoro-6-trifluoromethyl-2'H,3H-spiro[imidazo[1,2-a]pyridine-2,1'-naphthalene]

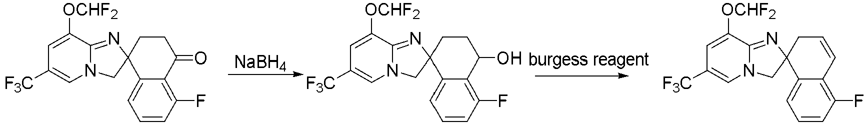

### Step 1: Synthesis of 8-Difluoromethoxy-5'-fluoro-6-trifluoromethyl-3',4'-dihydro-2'H,3H-spiro[imidazo[1,2-a]pyridine-2,1'-naphthalene]-4'-ol

8-Difluoromethoxy-5'-fluoro-6-trifluoromethyl-2',3'-dihydro-3'H,4'H-spiro[imidazo[1,2-a]pyridine-2,1'-naphthalene]-4'-one (60 mg) was dissolved in MeOH (2 mL), to which NaBH₄ (6 mg) was added. It was allowed to react at 0 °C for 0.5 h. Water was added, and the mixture was extracted, concentrated using a rotary evaporator, and purified by preparative TLC to afford the title compound (21 mg).

### Step 2: Synthesis of 8-Difluoromethoxy-5'-fluoro-6-trifluoromethyl-2'H,3H-spiro[imidazo[1,2-a]pyridine-2,1'-naphthalene]

8-Difluoromethoxy-5'-fluoro-6-trifluoromethyl-3',4'-dihydro-2'H,3H-spiro[imidazo[1,2-a]pyridine-2,1'-naphthalene]-4'-ol (21 mg) and Burgess reagent (37 mg) were dissolved in toluene (2 mL) and it was allowed to react at 90 °C for 0.5 h. Water was added, and the mixture was extracted, concentrated using a rotary evaporator, and purified by preparative HPLC to afford the title compound (4 mg). ESI-MS m/z: 387.31 [M+H]⁺; ¹H NMR (500 MHz, DMSO-d₆) δ 7.53-7.51 (m, 1H), 7.42 (t, 1H), 7.29-7.25 (m, 1H), 7.15-7.11 (m, 1H), 7.04 (ddd, 1H), 6.92 (d, 1H), 6.82 (dd, 1H), 4.31 (d, 1H), 3.79 (d, 1H), 2.65 (dt, 1H), 2.53 (dd, 1H).

### Example 16-A and Example 16-B: Preparation of (S)-8-Difluoromethoxy-5'-fluoro-6-trifluoromethyl-2'H,3H-spiro[imidazo[1,2-a]pyridine-2,1'-naphthalene] and (R)-8-Difluoromethoxy-5'-fluoro-6-trifluoromethyl-2'H,3H-spiro[imidazo[1,2-a]pyridine-2,1'-naphthalene]

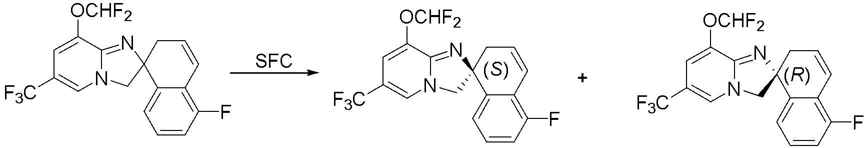

Racemic 8-difluoromethoxy-5'-fluoro-6-trifluoromethyl-2'H,3H-spiro[imidazo[1,2-a]pyridine-2,1'-naphthalene] (the compound of Example 16) was subjected to preparative SFC to obtain a pair of enantiomers. The enantiomer eluted first from the chiral column below was designated Example 16-A; the enantiomer eluted later was designated Example 16-B. (Note: The absolute configurations of the compounds of Example 16-A and 16-B have not been determined. For narrative convenience, the chiral center of Example 16-A is randomly assigned as S, and that of Example 16-B is assigned as R.)

Example 16-A: (S)-8-Difluoromethoxy-5'-fluoro-6-trifluoromethyl-2'H,3H-spiro[imidazo[1,2-a]pyridine-2,1'-naphthalene]: SFC (RT = 7.26 min, ee = 99.1%). Analytical method: DAICEL CHIRALPAK IG column (0.46 cm × 25 cm); mobile phase: ethanol/n-hexane (5:95, v/v) with 0.1% diethylamine; flow rate: 1 mL/min. ESI-MS m/z: 387.5 [M+H]⁺; ¹H NMR (500 MHz, DMSO-d₆) δ 7.88 (s, 1H), 7.45 (t, 1H), 7.29-7.25 (m, 1H), 7.14-7.09 (m, 1H), 7.08 (d, 1H), 7.04 (s, 1H), 6.75 (dd, 1H), 6.16 (ddd, 1H), 4.32 (d, 1H), 3.82 (d, 1H), 2.58-2.53 (m, 2H).

Example 16-B: (R)-8-Difluoromethoxy-5'-fluoro-6-trifluoromethyl-2'H,3H-spiro[imidazo[1,2-a]pyridine-2,1'-naphthalene]: SFC (RT = 7.82 min, ee = 96.1%). Analytical method: DAICEL CHIRALPAK IG column (0.46 cm × 25 cm); mobile phase: ethanol/n-hexane (5:95, v/v) with 0.1% diethylamine; flow rate: 1 mL/min. ESI-MS m/z: 387.5 [M+H]⁺; ¹H NMR (500 MHz, DMSO-d₆) δ 7.88 (s, 1H), 7.45 (t, 1H), 7.29-7.25 (m, 1H), 7.14-7.09 (m, 1H), 7.08 (d, 1H), 7.04 (s, 1H), 6.75 (dd, 1H), 6.16 (ddd, 1H), 4.32 (d, 1H), 3.82 (d, 1H), 2.58-2.53 (m, 2H).

### Example 17: Synthesis of 8-Difluoromethoxy-1'-methyl-6-trifluoromethyl-3H,6'H-spiro[imidazo[1,2-a]pyridine-2,5'-isoquinoline]

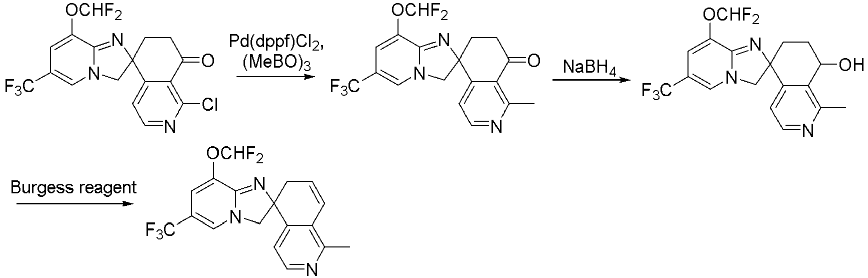

### Step 1: Synthesis of 8-Difluoromethoxy-1'-methyl-6-trifluoromethyl-6',7'-dihydro-3H,8'H-spiro[imidazo[1,2-a]pyridine-2,5'-isoquinoline]-8'-one

1'-Chloro-8-difluoromethoxy-6-trifluoromethyl-6',7'-dihydro-3H,8'H-spiro[imidazo[1,2-a]pyridine-2,5'-isoquinoline]-8'-one (100 mg) was dissolved in 1,4-dioxane (5 mL), to which H₂O (1 mL), Pd(dppf)Cl₂ (35.1 mg), and Cs₂CO₃ (156.4 mg) were added. After nitrogen purging, trimethylboroxine (0.7 mL) was added, and it was allowed to react at 100 °C for 13 h. Water was added, and the mixture was extracted, concentrated using a rotary evaporator, and purified by preparative TLC to afford the title compound (60 mg).

### Step 2: Synthesis of 8-Difluoromethoxy-1'-methyl-6-trifluoromethyl-7',8'-dihydro-3H,6'H-spiro[imidazo[1,2-a]pyridine-2,5'-isoquinoline]-8'-ol

8-Difluoromethoxy-1'-methyl-6-trifluoromethyl-6',7'-dihydro-3H,8'H-spiro[imidazo[1,2-a]pyridine-2,5'-isoquinoline]-8'-one (38 mg) was dissolved in MeOH (3 mL) and cooled to 0 °C. NaBH₄ (3.6 mg) was added slowly, and the mixture was stirred for 30 min. Water was added, and the mixture was extracted, concentrated using a rotary evaporator, and purified by preparative TLC to afford the title compound (12 mg).

### Step 3: Synthesis of 8-Difluoromethoxy-1'-methyl-6-trifluoromethyl-3H,6'H-spiro[imidazo[1,2-a]pyridine-2,5'-isoquinoline]

8-Difluoromethoxy-1'-methyl-6-trifluoromethyl-7',8'-dihydro-3H,6'H-spiro[imidazo[1,2-a]pyridine-2,5'-isoquinoline]-8'-ol (12 mg) was dissolved in PhMe (2 mL), to which Burgess reagent (21.5 mg) was added. After nitrogen purging, it was allowed to react at 100 °C for 1 h. Water was added, and the mixture was extracted, concentrated using a rotary evaporator, and purified by preparative TLC to afford the title compound, Example 17 (5 mg). ESI-MS m/z: 384.39 [M+H]⁺; ¹H NMR (500 MHz, Acetonitrile-d₃) δ 8.20 (d, 1H), 7.41-7.39 (m, 1H), 7.13 (t, 1H), 6.98 (s, 1H), 6.81 (d, 1H), 6.69 (dd, 1H), 6.09-6.04 (m, 1H), 4.20 (d, 1H), 3.66 (d, 1H), 2.52 (dt, 1H), 2.44 (s, 3H), 2.42 (dd, 1H).

### Example 18: Synthesis of 8-Difluoromethoxy-5'-fluoro-6'-methyl-6-trifluoromethyl-2'H,3H-spiro[imidazo[1,2-a]pyridine-2,1'-naphthalene]

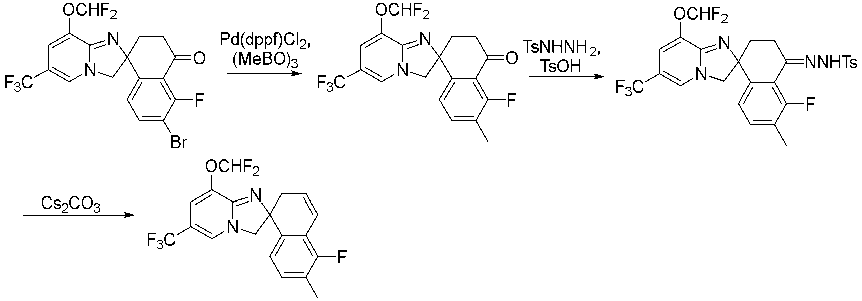

### Step 1 : Synthesis of 8-Difluoromethoxy-5'-fluoro-6'-methyl-6-trifluoromethyl-2',3'-dihydro-3H,4'H-spiro[imidazo[1,2-a]pyridine-2,1'-naphthalene]-4'-one

6'-Bromo-8-difluoromethoxy-5'-fluoro-6-trifluoromethyl-2',3'-dihydro-3H,4'H-spiro[imidazo[1,2-a]pyridine-2,1'-naphthalene]-4'-one (80 mg), Pd(dppf)Cl₂ (24.3 mg), and Cs₂CO₃ (108.2 mg) were added to 1,4-dioxane (3 mL) and H₂O (0.6 mL) for dissolution. After nitrogen purging, trimethylboroxine (0.47 mL) was added, and it was allowed to react at 100 °C for 1 h. Water was added, and the mixture was extracted, concentrated using a rotary evaporator, and purified by preparative TLC to afford the title compound (59 mg).

### Step 2: Synthesis of N'-(8-Difluoromethoxy-5'-fluoro-6'-methyl-6-trifluoromethyl-2',3'-dihydro-3H,4'H-spiro[imidazo[1,2-a]pyridine-2,1'-naphthalene]-4'-ylidene)-4-toluenesulfonylhydrazone

8-Difluoromethoxy-5'-fluoro-6'-methyl-6-trifluoromethyl-2',3'-dihydro-3H,4'H-spiro[imidazo[1,2-a]pyridine-2,1'-naphthalene]-4'-one (59.4 mg), TsNHNH₂ (40 mg), and TsOH (29.6 mg) were added to THF (5 mL). After nitrogen purging, it was allowed to react at 40 °C for 2 h. Water was added, and the mixture was extracted, concentrated using a rotary evaporator, and purified by preparative TLC to afford the title compound (97 mg).

### Step 3: Synthesis of 8-Difluoromethoxy-5'-fluoro-6'-methyl-6-trifluoromethyl-2'H,3H-spiro[imidazo[1,2-a]pyridine-2,1'-naphthalene]

N'-(8-Difluoromethoxy-5'-fluoro-6'-methyl-6-trifluoromethyl-2',3'-dihydro-3H,4'H-spiro[imidazo[1,2-a]pyridine-2,1'-naphthalene]-4'-ylidene)-4-toluenesulfonylhydrazone (40 mg) and Cs₂CO₃ (66.8 mg) were added to 1,4-dioxane (3 mL) and the mixture was stirred at 90 °C for 5 h. Water was added, and the mixture was extracted, concentrated using a rotary evaporator, and purified by preparative TLC to afford the title compound, Example 18 (13 mg). ESI-MS m/z: 401.13 [M+H]⁺; ¹H NMR (500 MHz, DMSO-d₆) δ 7.87 (s, 1H), 7.44 (t, 1H), 7.14 (t, 1H), 7.02 (s, 1H), 6.95 (d, 1H), 6.74 (d, 1H), 6.16-6.10 (m, 1H), 4.28 (d, 1H), 3.79 (d, 1H), 2.57-2.52 (m, 1H), 2.49-2.46 (m, 1H), 2.21 (s, 3H).

### Example 19: Synthesis of 8-Difluoromethoxy-5'-fluoro-4'-iodo-6-trifluoromethyl-2'H,3H-spiro[imidazo[1,2-a]pyridine-2,1'-naphthalene]

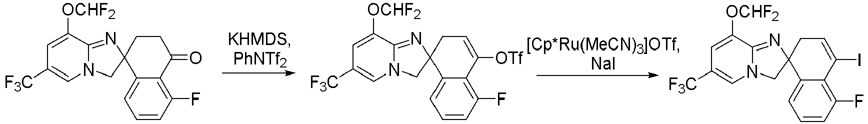

### Step 1: Synthesis of 8-Difluoromethoxy-5'-fluoro-6-trifluoromethyl-2'H,3H-spiro[imidazo[1,2-a]pyridine-2,1'-naphthalene]-4'-yl trifluoromethanesulfonate

8-Difluoromethoxy-5'-fluoro-6-trifluoromethyl-2',3'-dihydro-3H,4'H-spiro[imidazo[1,2-a]pyridine-2,1'-naphthalene]-4'-one (500 mg) was dissolved in THF (4 mL). After nitrogen purging, the mixture was cooled to -78 °C, to which KHMDS (1.5 mL) was added dropwise, and it was allowed to react for 1 hour. PhNTf₂ was dissolved in THF (2 mL) and then was added dropwise to the reaction mixture. It was allowed to react 30 minutes. The reaction was quenched with saturated NH₄Cl solution, and the resulting mixture was extracted, concentrated using a rotary evaporator, and purified by silica gel column chromatography to afford the title compound (306 mg).

### Step 2: Synthesis of 8-Difluoromethoxy-5'-fluoro-4'-iodo-6-trifluoromethyl-2'H,3H-spiro[imidazo[1,2-a]pyridine-2,1'-naphthalene]

8-Difluoromethoxy-5'-fluoro-6-trifluoromethyl-2'H,3H-spiro[imidazo[1,2-a]pyridine-2,1'-naphthalene]-4'-yl trifluoromethanesulfonate (135 mg), NaI (120 mg), and [Cp*Ru(MeCN)₃]OTf (15 mg) were dissolved in DMI (2 mL). After nitrogen purging, the mixture was heated to 100 °C and it was allowed to react at that temperature for 16 hours. Water was added, and the mixture was extracted, concentrated using a ratary evaporator, and purified by silica gel column chromatography to afford the title compound (117 mg). ESI-MS m/z: 513.20 [M+H]⁺; ¹H NMR (500 MHz, DMSO-d₆) δ 7.90 (s, 1H), 7.44 (t, 1H), 7.39 (td, 1H), 7.17 (ddd, 1H), 7.10 (dd, 1H), 7.06 (s, 1H), 6.99 (dd, 1H), 4.42 (d, 1H), 3.87 (d, 1H), 2.54 (dd, 1H), 2.40 (dd, 1H).

### Example 20: Synthesis of 8-Difluoromethoxy-6-trifluoromethyl-3H,6'H-spiro[imidazo[1,2-a]pyridine-2,5'-isoquinoline]

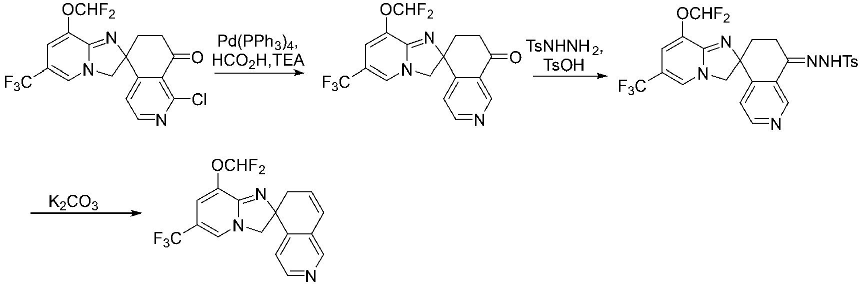

### Step 1: Synthesis of 8-Difluoromethoxy-6-trifluoromethyl-6',7'-dihydro-3H,8'H-spiro[imidazo[1,2-a]pyridine-2,5'-isoquinoline]-8'-one

1'-Chloro-8-difluoromethoxy-6-trifluoromethyl-6',7'-dihydro-3H,8'H-spiro[imidazo[1,2-a]pyridine-2,5'-isoquinoline]-8'-one (100 mg) was dissolved in N,N-dimethylacetamide (3 mL), to which Pd(PPh₃)₄ (27.7 mg), formic acid (16.6 mg), and TEA (38.5 mg) were added. After nitrogen purging, the mixture was heated to 100 °C and it was allowed to react at that temperature for 5 hours. Water was added, and the mixture was extracted, concentrated using a rotary evaporator, and purified by silica gel column chromatography to afford the title compound (90 mg).

### Step 2: Synthesis of N'-(8-Difluoromethoxy-6-trifluoromethyl-6',7'-dihydro-3H,8'H-spiro[imidazo[1,2-a]pyridine-2,5'-isoquinoline]-8'-ylidene)-4-methylbenzene sulfonylhydrazone

8-Difluoromethoxy-6-trifluoromethyl-6',7'-dihydro-3H,8'H-spiro[imidazo[1,2-a]pyridine-2,5'-isoquinoline]-8'-one (40 mg), TsNHNH₂ (29 mg), and TsOH (2 mg) were added to THF (2 mL). After nitrogen purging, it was allowed to react at 30 °C for 4 h. Water was added, and the mixture was extracted, concentrated using a rotary evaporator, and purified by preparative TLC to afford the title compound (48 mg).

### Step 3: Synthesis of 8-Difluoromethoxy-6-trifluoromethyl-3H,6'H-spiro[imidazo[1,2-a]pyridine-2,5'-isoquinoline]

N'-(8-Difluoromethoxy-6-trifluoromethyl-6',7'-dihydro-3H,8'H-spiro[imidazo[1,2-a]pyridine-2,5'-isoquinoline]-8'-ylidene)-4-methylbenzene sulfonyl hydrazone (38 mg) was dissolved in 1,4-dioxane (3 mL), to which K₂CO₃ (32 mg) was added. After nitrogen purging, it was allowed to react at 90 °C for 16 h. Water was added, and the mixture was extracted, concentrated using a rotary evaporator, and purified by preparative TLC to afford the title compound, Example 20 (9.7 mg). ESI-MS m/z: 369.92 [M+H]⁺; ¹H NMR (500 MHz, Acetonitrile-d₃) δ 8.44 (d, J = 5.0 Hz, 1H), 8.37 (s, 1H), 7.54-7.51 (m, 1H), 7.24 (t, 1H), 7.24 (d, 1H), 6.94 (d, 1H), 6.66 (dd, 1H), 6.17 (ddd, 1H), 4.31 (d, 1H), 3.83 (d, 1H), 2.67 (dt, 1H), 2.57 (dd, 1H).

### Biological Assays

### Determination of EPO Inhibition Rate by Compounds Using Hep3B Cells

Hep3B cells (from the Cell Bank of the Chinese Academy of Sciences, TCHu106) were seeded in a plate at a density of 5×10⁴/mL and incubated overnight at 37°C in a 5% CO₂ incubator (Thermo Scientific BB150). The next day, the culture medium was replaced with medium containing 0.5% FBS (VivaCell C04001-500) to starve the cells for 6 hours. Subsequently, a DMSO solution of (6'-hydroxy-3'-(4-methoxyphenyl)-8'-oxo-8'H-spiro[cyclopentane-1,5'-indolizine]-7'-carbonyl)glycine or its analogues was added to achieve a final concentration of 30 µM. The plate was returned to the 37°C, 5% CO₂ incubator for an additional 24 hours. Then, a DMSO solution of the test compound was added to achieve final concentrations of 30000 nM, 7500 nM, 1875 nM, 469 nM, 117 nM, 29 nM, 7.3 nM, 1.8 nM, 0.5 nM, and 0 nM (ten concentrations in total). Treatment continued for 48 hours. Cell culture supernatants were collected for EPO ELISA detection. The assay was performed according to the instructions of the ELISA kit (Sangon Biotech (Shanghai) Co., Ltd., D711311-0096). Absorbance was read at a wavelength of 450 nm using a microplate reader (Thermo Scientific Multiskan Go, 511191200).

The compound inhibition rate was calculated using the formula: Inhibition Rate = 1 - 100% * (EPO value of the treated well - EPO value of the blank well) / (EPO value of the Control well - EPO value of the blank well). The IC₅₀ value was calculated by linear fitting of the inhibition rates. Herein, the Control well refers to the cell-containing well treated for 72 hours with a DMSO solution containing 0 nM of the test compound but containing 30 µM of (6'-hydroxy-3'-(4-methoxyphenyl)-8'-oxo-8'H-spiro[cyclopentane-1,5'-indolizine]-7'-carbonyl)glycine or its analogues. The EPO level in this well was set as 100%. The blank well refers to a cell-containing well without the addition of any compound.

The IC₅₀ data for the positive control compound and certain example compounds were provided in Table 1. (Due to instrumental errors and variations in operator techniques, the measured IC₅₀ values of compounds under different experimental conditions may exhibit some discrepancies. Therefore, the values listed in the table should be understood as indicating relative magnitudes within a certain margin of error.)

As shown in Table 1, compared with the positive control compound, all tested example compounds exhibited IC₅₀ values comparable to or lower than that of the positive control compound. This indicates that the tested example compounds possess excellent activity in reducing EPO levels, thereby demonstrating potential for use in the treatment of EPO-related diseases.

**Table 1**

| **Compounds** | | **IC₅₀ (nM)** |
|---|---|---|
| Example 1 | | 29.0 |
| Example 1-A | | 17.9 |
| Example 1-B | | 563 |
| Example 2 | | 35.8 |
| Example 2-A | | 365 |
| Example 2-B | | 18.7 |
| Example 6 | | 181 |
| Example 7 | | 24.4 |
| Example 8 | | 148 |
| Example 9 | | 47.9 |
| Example 10 | | 26.8 |
| Example 10-A | | 2152 |
| Example 10-B | | 26.9 |
| Example 11 | | 12.2 |
| Example 12 | | 1.08 |
| Example 13 | | 116 |
| Example 14 | | 4.12 |
| Example 15 | | 3.04 |
| Example 15-A | | 2.12 |
| Example 15-B | | 109 |
| Example 16 | | 7.01 |
| Example 16-A | | 6.45 |
| Example 16-B | | 20.4 |
| Example 17 | | 705 |
| Example 18 | | 3.48 |
| Example 19 | | 302 |
| Example 20 | | 813 |
| positive control compound as prepared according to J. Med. Chem. 2013, 56, 1739-1747 | 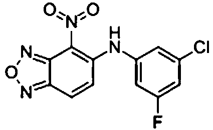 | 497 |

Although specific embodiments of the present disclosure have been illustrated and described, it does not mean that these embodiments illustrate and describe all possible implementation forms of the present disclosure. More precisely, the language used in this specification are only descriptive words and not restrictive. It will be obvious to those skilled in the art that various kinds of changes and modifications can be made without departing from the general scope of the present disclosure. Therefore, the appended claims are intended to include all these changes and modifications within the scope of the present disclosure.

## Claims

1. A compound of formula (I), its isotopically labeled compound, its optical isomers, its tautomers, or its pharmaceutically acceptable salts thereof, or crystalline forms or solvates of the compound or its pharmaceutically acceptable salts, 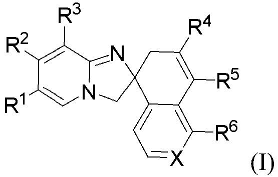 wherein
R¹ is selected from H, D, F, Cl, Br, CF₃, and CN;
R² is selected from H, D, CF₃, CH₃, OCH₃, and OH;
R³ is selected from H, D, F, Cl, Br, CF₃, CN, OCH₃, OCH₂F, OCHF₂, and OCF₃;
R⁴ is selected from H, D, F, Cl, Br, CH₃, CF₃, and CN;
R⁵ is selected from H, D, F, Cl, Br, I, CH₃, CF₃, CN, and OCH₃;
R⁶ is selected from H, D, F, Cl, Br, I, CH₃, CF₃, CN, and OCH₃; and
X is selected from N, CR⁷; wherein R⁷ is selected from H, D, F, Cl, Br, CF₃, CN, and CH₃.

2. The compound of formula (I), its isotopically labeled compound, its optical isomers, its tautomers, or its pharmaceutically acceptable salts thereof, or crystalline forms or solvates of the compound or its pharmaceutically acceptable salts as claimed in claim 1, wherein R¹ is selected from CF₃, and CN.

3. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 2, wherein R² is selected from OCH₃, and OH.

4. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 3, wherein R³ is selected from OCH₃, OCH₂F, OCHF₂, and OCF₃.

5. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 4, wherein R⁴ is selected from H, D, F, Cl, and CH₃.

6. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 5, wherein R⁵ is selected from H, D, F, Cl, CH₃, CF₃, and OCH₃.

7. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 6, wherein R⁶ is selected from F, Cl, and CH₃.

8. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 8, wherein X is selected from N, CR⁷; wherein R⁷ is selected from H, D, F, Br, CF₃, CN, and CH₃.

9. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 8, wherein R¹ is CF₃; R² is selected from OCH₃, OH, and H; R³ is OCH₂F; R⁴ is selected from H, F, Cl, and CH₃; R⁵ is selected from H, F, Cl, I, CH₃, CF₃, and OCH₃; R⁶ is selected from F, Cl, and CH₃; and X is selected from N and CR⁷, wherein R⁷ is selected from H, F, and Br.

10. The compound of formula (I), its isotopically labeled compound, its optical isomers, its tautomers, or its pharmaceutically acceptable salts thereof, or crystalline forms or solvates of the compound or its pharmaceutically acceptable salts as claimed in any one of claims 1 to 8, wherein R¹ is CF₃; R² is selected from OCH₃, OH, and H; R³ is OCH₂F or H; R⁴ is selected from H, F, Cl, and CH₃; R⁵ is selected from H, F, Cl, Br, I, CH₃, CF₃, and OCH₃; R⁶ is selected from H, F, Cl, and CH₃; and X is selected from N and CR⁷, wherein R⁷ is selected from H, F, Br and CH₃.

11. The compound of formula (I), its isotopically labeled compound, its optical isomers, its tautomers, or its pharmaceutically acceptable salts thereof, or crystalline forms or solvates of the compound or its pharmaceutically acceptable salts as claimed in claim 1, wherein the compound is selected from:

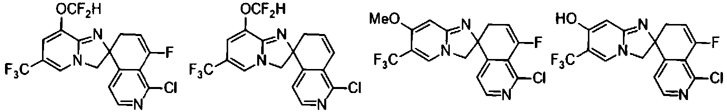

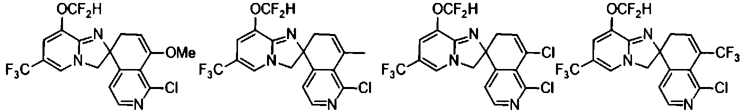

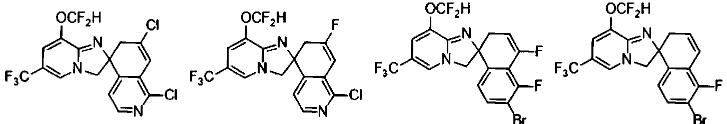

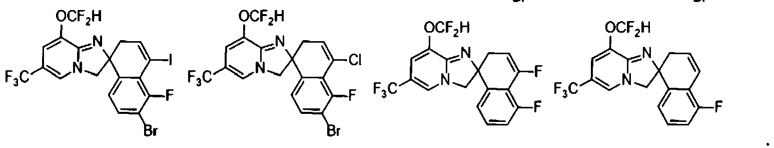

12. The compound of formula (I), its isotopically labeled compound, its optical isomers, its tautomers, or its pharmaceutically acceptable salts thereof, or crystalline forms or solvates of the compound or its pharmaceutically acceptable salts as claimed in claim 1, wherein the compound is selected from:

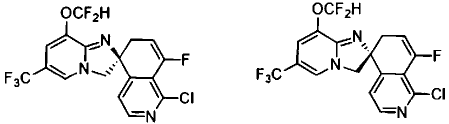

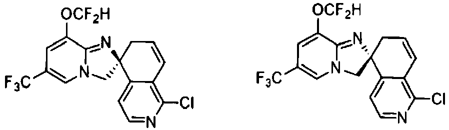

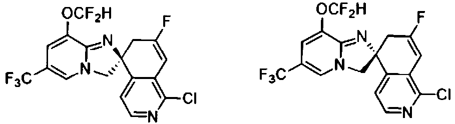

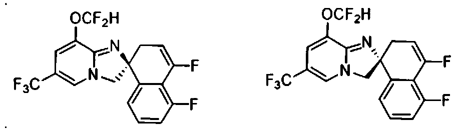

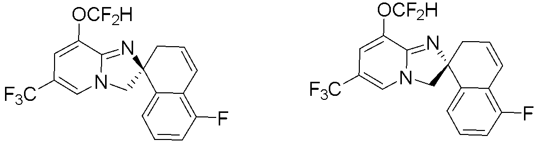

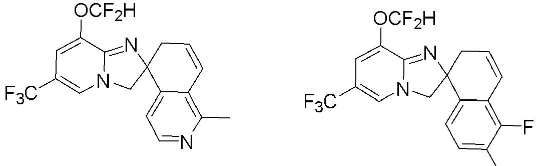

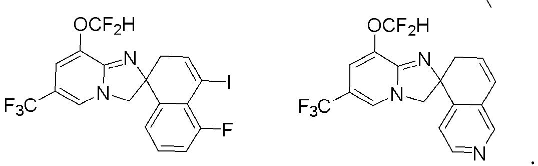

13. A pharmaceutical composition, comprising the compound, its isotopically labeled compound, its optical isomers, its tautomers, or its pharmaceutically acceptable salts thereof, or crystalline forms or solvates of the compound or its pharmaceutically acceptable salts as claimed in any one of claims 1 to 12, and one or more pharmaceutically acceptable carriers, adjuvants or excipients.

14. Use of a compound, its isotopically labeled compound, its optical isomers, its tautomers, or its pharmaceutically acceptable salts thereof, or crystalline forms or solvates of the compound or its pharmaceutically acceptable salts as claimed in any one of claims 1 to 12 or a pharmaceutical composition as claimed in claim 13, in the manufacture of a medicament for treating a disease or disorder associated with EPO.

15. Use according to claim 14, wherein the diseases and disorders associated with EPO are selected from: polycythemia, renal cell carcinoma and other cancers, including but not limited to bladder cancer, breast cancer, cervical cancer, colorectal cancer, small intestinal cancer, colon cancer, rectal cancer, anal cancer, endometrial cancer, head and neck cancer, liver cancer, lung cancer, ovarian cancer, prostate cancer, testicular cancer, uterine cancer, esophageal cancer, gallbladder cancer, pancreatic cancer, thyroid cancer, parathyroid cancer, skin cancer, and brain cancer.
